# FASCICULE DE BREVET EUROPEEN

(11) **EP 0 882 033 B1**
(45) Date de publication et mention de la délivrance du brevet: **16.08.2001**
(21) Numéro de dépôt: 97950223.4
(22) Date de dépôt: 04.12.1997
(51) Int. Cl.: C07D 307/79, C07D 307/83, C07D 307/86, C07D 307/78, C07D 333/54, C07D 333/52, C07D 209/08, C07D 209/04, A61K 31/34, A61K 31/38, A61K 31/40, A61K 7/42, A61K 7/06, A61K 7/48

(54) **DERIVES D'ACIDES BENZOFURANNE-ACRYLIQUES ET LEUR UTILISATION COMME MODULATEURS DES RECEPTEURS RXRS OU RARS**
BENZOFURAN-ACRYLSAURE-DERIVATE UND IHRE ANWENDUNG ALS MODULATOREN DER RXRS ODER RARS REZEPTOREN
BENZOFURAN-ACRYLIC ACID DERIVATIVES AND THEIR USE AS MODULATORS OF RXRS OR RARS RECEPTORS

(30) Priorité: 04.12.1996 FR 9614882
(43) Date de publication de la demande: 09.12.1998
(73) Titulaire: CENTRE INTERNATIONAL DE RECHERCHES DERMATOLOGIQUES GALDERMA, ( CIRD GALDERMA), 06560 Valbonne (FR)
(72) Inventeur: DIAZ, Philippe, route de Saint-Antoine F-06200 Nice (FR); CHARPENTIER, Bruno, F-06410 Biot (FR)
(74) Mandataire: Tezier Herman, Béatrice
(86) Numéro de dépôt international: FR9702205
(87) Numéro de publication internationale: WO9824778

(56) Documents cités:
- EP-A- 0 568 898
- WO-A-96/13478
- WO-A-97/29100
- US-A- 4 326 055

## Description

L'invention concerne, à titre de produits industriels nouveaux et utiles, des composés biaromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit'photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

La présente invention concerne des composés qui peuvent être représentés par la formule générale (I) suivante : 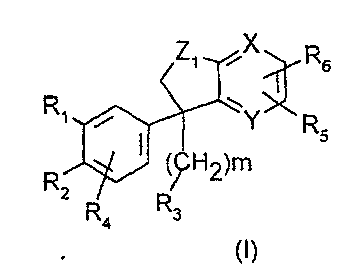 dans laquelle :
Z₁ représente un atome ou un radical choisi parmi : O, S et NR',
X et Y, identiques ou différents, représentent CH ou N, étant entendu que X et Y ne peuvent être simultanément des atomes d'azote,
R₁ et R₂ pris ensemble forment avec le cycle aromatique adjacent un cycle à 5 ou 6 chainons éventuellement substitué par des groupes méthyle et /ou éventuellement interrompu par un un radical SO, un radical SO₂, un atome d'oxygène ou de soufre,
R₃ représente :
   (i) un atome d'hydrogène, un radical alkyle ayant de 1 à 6 atomes de carbone linéaire ou ramifié éventuellement substitué par un ou plusieurs atomes d'halogène , un radical alkényle ayant de 2 à 6 atomes de carbone linéaire ou ramifié, un radical alkynyle ayant de 3 à 6 atomes de carbone linéaire ou ramifié, un radical aryle, un radical monohydroxyalkyle ou polyhydroxyalkyle, un radical polyéther, un radical cyano ou un radical -O-R₇,
      R₇ ayant la signification donnée ci-après,
   (ii) un radical de formule : 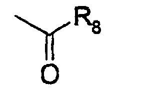 R₈ ayant la signification donnée ci-après,
   ou (iii) un radical de formule : 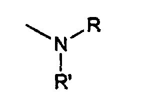 R et R' ayant la signification donnée ci-après
R₄ représente :
   (i) un atome d'hydrogène,
   (ii) un radical alkyle ayant de 1 à 6 atomes de carbone linéaire ou ramifié éventuellement substitué par un ou plusieurs atomes d'halogène ,
   (iii) un atome d'halogène,
   (iv) un radical -OR₇,
      R₇ ayant la signification donnée ci-après,
R₅ représente :
   (i) un radical de formule : 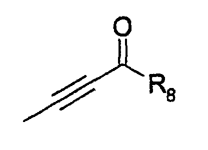
   (ii) un radical de formule : 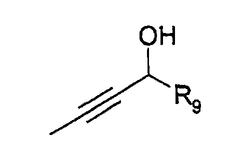
   (iii) un radical de formule : 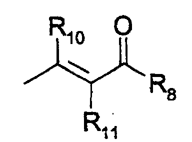
   (iv) un radical de formule : 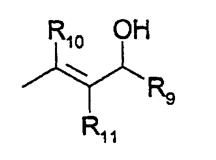
   (v) un radical de formule : 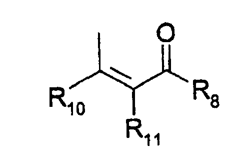
   (vi) un radical de formule : 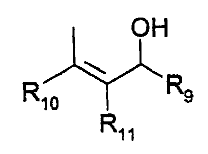 R₈, R₉, R₁₀ et R₁₁ ayant les significations données ci-après,
R₆ représente un atome d' hydrogène, un atome d' halogène, un radical alkyle ayant de 1 à 6 atomes de carbone linéaire ou ramifié éventuellement substitué par un ou plusieurs atomes d'halogène ou le radical -OR₇,
   R₇ ayant la signification donnée ci-après,
R₇, identique ou différent, représente un atome d'hydrogène, un radical alkyle ayant de 1 à 6 atomes de carbone linéaire ou ramifié éventuellement substitué par un ou plusieurs atomes d'halogène , un radical aryle, un radical aralkyle, éventuellement substitué(s), un radical monohydroxyalkyle ou polyhydroxyalkyle, un radical polyéther ou un radical acyle ayant de 1 à 6 atomes de carbone linéaire ou ramifié,
   R₈, identique ou différent, représente :
   (a) un atome d'hydrogène, un radical alkyle ayant de 1 à 6 atomes de carbone linéaire ou ramifié éventuellement substitué par un ou plusieurs atomes d'halogène ,
   (b) un radical de formule : 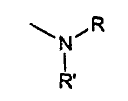 R et R' ayant la signification donnée ci-après,
   (c) un radical -OR₁₂
   (d) un reste de sucre ou d'aminoacide,
R₉, identique ou différent, représente un atome d'hydrogène ou un radical alkyle ayant de 1 à 6 atomes de carbone linéaire ou ramifié éventuellement substitué par un ou plusieurs atomes d'halogène ,
R₁₀ et R₁₁, identiques ou différents, représentent un atome d'hydrogène ou un radical alkyle ayant de 1 à 6 atomes de carbone linéaire ou ramifié éventuellement substitué par un ou plusieurs atomes d'halogène ,
R₁₂ représente un atome d'hydrogène, un radical alkyle ayant de 1 à 6 atomes de carbone linéaire ou ramifié éventuellement substitué par un ou plusieurs atomes d'halogène, un radical mono- ou polyhydroxyalkyle, un radical aryle ou aralkyle éventuellement substitué(s),
R et R', identiques ou différents, représentent des groupements protecteurs de fonctions amines, un atome d'hydrogène, un radical alkyle ayant de 1 à 6 atomes de carbone linéaire ou ramifié éventuellement substitué par un ou plusieurs atomes d'halogène ou encore, pris ensemble, forment un hétérocycle,
m est égal à 0 ou 1,
et les isomères optiques desdits composés de formule (I) ainsi que leurs sels.

Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisucinique, maléique et mandélique. Lorsque les composés selon l'invention se présentent sous forme de sels par addition d'une base, il s'agit préférentiellement de sels d'un métal alcalin ou alcalino-terreux ou encore de zinc ou d'une amine organique.

Parmi les radicaux alkyle ayant de 1 à 6 atomes de carbone linéaire ou ramifié éventuellement substitué par un ou plusieurs atomes d'halogène, on peut plus particulièrement citer les radicaux méthyle, éthyle, isopropyle, butyle, tertiobutyle et hexyle.

Parmi les radicaux alkényle ayant de 2 à 6 atomes de carbone linéaire ou ramifié on préfère un radical ayant de 2 à 6 atomes de carbone linéaire ou ramifié comportant une ou plusieurs doubles liaisons et de préférence les radicaux allyle ou vinyle.

Parmi les radicaux alkynyle ayant de 3 à 6 atomes de carbone linéaire ou ramifiés, on entend un radical ayant de 3 à 6 atomes de carbone linéaire ou ramifié comportant une à plusieurs triple liaisons. On peut plus particulièrement citer le radical propargyle.

Par radical acyle ayant de 1 à 6 atomes de carbone linéaire ou ramifié, on entend un radical ayant de 1 à 6 atomes de carbone et, de préférence, les radicaux acétyle, propionyle ou pivaloyle.

Par groupement protecteur de fonction amine, on entend les groupements correspondants décrits dans "Protecting groups in organic synthesis" by T.W Greene, Ed. by John Wiley and Sons (1981).

Par radical polyéther, on entend un radical ayant de 1 à 6 atomes de carbone et de 1 à 3 atomes d'oxygène ou de soufre, tel que les radicaux méthoxyméthyl éther, méthoxyéthoxyméthyl éther ou méthylthiométhyl éther.

Par radical monohydroxyalkyle ou polyhydroxyalkyle, on doit entendre un radical contenant de 1 à 6 atomes de carbone et de 1 à 5 groupes hydroxyles.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, éventuellement substitués, on préfère un radical phényle, éventuellement substitué par un ou plusieurs atomes d'halogène, une fonction hydroxyle ou fonction nitro, un groupe méthoxy ou une fonction amine éventuellement substituée.

Parmi les radicaux aralkyle, éventuellement substitués, on préfère le radical benzyle ou phénéthyle, éventuellement substitué par un ou plusieurs atomes d'halogène, une fonction hydroxyle ou nitro, ou un groupe méthoxy.

Parmi les restes d'aminoacide, on préfère un reste dérivant par exemple d'au moins un des 20 aminoacides de configuration L ou D constitutifs de protéines de mammifères. Ils sont plus particulièrement choisis parmi les restes dérivant de la lysine, de la glycine ou de l'acide aspartique.

Parmi les restes de sucre, on préfère un reste dérivant par exemple du glucose, du galactose, du mannose ou de l'acide glucuronique.

Parmi les hétérocycles, on préfère un radical pipéridino, morpholino, pyrrolidino ou pipérazino, éventuellement substitué en position 4 par un radical alkyle en C₁-C₆ ou par un mono- ou polyhydroxyalkyle tels que définis ci-dessus.

Lorsque les radicaux R₄ et R₆ représentent un atome d'halogène, celui-ci est de préférence un'atome de fluor, de brome ou de chlore. Il en est de même pour les radicaux définis ci-dessus substitués par un ou des atomes d'halogène.

Parmi les composés de formule (I) ci-dessus rentrant dans le cadre de la présente invention, on peut notamment citer les suivants :
3-[3-(5,6,7,8-tetrahydro-5,5,8,8-tetraméthyl-2-naphtyl)-3-méthyl-2,3-dihydrobenzofuran-5-yl]-acrylate d'éthyle,
acide 3-[3-(5,6,7,8-tetrahydro-5,5,8,8-tetraméthyl-2-naphtyl)-3-méthyl-2,3-dihydrobenzofuran-5-yl]-acrylique,
acide [3-méthyl-3-(5,5,8,8-tetraméthyl-5,6,7,8-tetrahydro-naphtalen-2-yl)-2,3-dihydro-benzofuran-5-yl]-propynoique.
(+)-3-[3-méthyl-3-(5,5,8,8-tetraméthyl-5,6,7,8-tetrahydro-naphtalen-2-yl)-2,3-dihydro-benzofuran-5-yl]-acrylate d'éthyle
acide (+)-3-[3-méthyl-3-(5,5,8,8-tetraméthyl-5,6,7,8-tetrahydro-naphtalen-2-yl)-2,3-dihydro-benzofuran-5-yl]-acrylique
(-)-3-[3-méthyl-3-(5,5,8,8-tetraméthyl-5,6,7,8-tetrahydro-naphtalen-2-yl)-2,3-dihydro-benzofuran-5-yl]-acrylate d'éthyle
acide (-)-3-[3-méthyl-3-(5,5,8,8-tetraméthyl-5,6,7,8-tetrahydro-naphtalen-2-yl)-2,3-dihydro-benzofuran-5-yl]-acrylique.
3-[3-méthyl-3-(3,5,5,8,8-pentaméthyl-5,6,7,8-tetrahydro-naphtalen-2-yl)-2,3-dihydro-benzofuran-5-yl]-acrylate d'éthyle
acide 3-[3-méthyl-3-(3,5,5,8,8-pentaméthyl-5,6,7,8-tetrahydro-naphtalen-2-yl)-2,3-dihydro-benzofuran-5-yl]-acrylique.
3-[3-méthyl-3-(naphtalen-2-yl)-2,3-dihydro-benzofuran-5-yl]-acrylate d'éthyle
acide 3-[3-méthyl-3-(naphtalen-2-yl)-2,3-dihydro-benzofuran-5-yl]-acrylique. 3-[3-(8,8-diméthyl-5,6,7,8-tetrahydro-naphtalen-2-yl)-3-méthyl-2,3-dihydrobenzofuran-5-yl]-acrylate d'éthyle
acide 3-[3-(8,8-diméthyl-5,6,7,8-tetrahydro-naphtalen-2-yl)-3-méthyl-2,3-dihydrobenzofuran-5-yl]-acrylique
3-[3-(5,5-diméthyl-5,6,7,8-tetrahydro-naphtalen-2-yl)-3-méthyl-2,3-dihydrobenzofuran-5-yl]-acrylate d'éthyle.
acide 3-[3-(5,5-diméthyl-5,6,7,8-tetrahydro-naphtalen-2-yl)-3-méthyl-2,3-dihydrobenzofuran-5-yl]-acrylique.
3-[3-méthyl-3-(5,5,8,8-tetraméthyl-5,6,7,8-tetrahydro-naphtalen-2-yl)-2,3-dihydrobenzofuran-6-yl]-acrylate d'éthyle.
acide 3-[3-méthyl-3-(5,5,8,8-tetraméthyl-5,6,7,8-tetrahydro-naphtalen-2-yl)-2,3-dihydro-benzofuran-6-yl]-acrylique.
3-[3-allyl-3-(5,5,8,8-tetramélhyl-5,6,7,8-tetrahydro-naphtalen-2-yl)-2,3-dihydro-benzofuran-5-yl]-acrylate d'éthyle
acide 3-[3-allyl-3-(5,5,8,8-tetraméthyl-5,6,7,8-tetrahydro-naphtalen-2-yl)-2,3-dihydro-benzofuran-5-yl]-acrylique
(E)-3-[3-méthyl-3-(5,5,8,8-tetraméthyl-5,6,7,8-tetrahydro-naphtalen-2-yl)-2,3-dihydro-benzofuran-5-yl]-but-2 enoate de méthyle
(Z)-3-[3-méthyl-3-(5,5,8,8-tetraméthyl-5,6,7,8-tetrahydro-naphtalen-2-yl)-2,3-dihydro-benzofuran-5-yl]-but-2-enoate de méthyle.
acide (E)-3-[3-méthyl-3-(5,5,8,8-tetraméthyl-5,6,7,8-tetrahydro-naphtalen-2-yl)-2,3-dihydro-benzofuran-5-yl]-but-2-enoique
3-[3-méthyl-3-(5,5,8,8-tetraméthyl-5,6,7,8-tetrahydro-naphtalen-2-yl)-2,3-dihydrobenzo[b]thiophen-5-yl]-acrylate d'éthyle.
acide 3-[3-méthyl-3-(5,5,8,8-tetraméthyl-5,6,7,8-tetrahydro-naphtalen-2-yl)-2,3-dihydro-benzo[*b*]thiophen-5-yl]-acrylique.
3-[3-(5,6,7,8-tetrahydro-5,5,8,8-tetraméthyl-2-naphtyl)-7-méthoxy-3-méthyl-2,3-dihydro-benzafuran-5-yl]-acrylate d'éthyle
acide 3-[3-(5,6,7,8-tetrahydro-5,5,8,8-tetraméthyl-2-naphtyl)-7-méthoxy-3-méthyl-2,3-dihydro-benzofuran-5-yl]-acrylique.
*N*-(4-hydroxy-phenyl)-3-[7-methoxy-3-méthyl-3-(5,5,8,8-tetraméthyl-5,6,7,8-tetrahydro-naphtalen-2-yl)-2,3-dihydro-benzofuran-5-yl]-acrylamide.
acide 3-[3-méthyl-3-(5,5,8,8-tetraméthyl-5,6,7,8-tetrahydro-naphtalen-2-yl)-2,3-dihydro-*1H*-indol-5-yl]-acrylique.
3-[3-méthyl-3-(5,5,8,8-tetraméthyl-5,6,7,8-tetrahydro-naphtalen-2-yl)-2,3-dihydro-*1H*-indol-5-yl]-acrylate de méthyle.

Selon la présente invention, les composés de formule (I) plus particulièrement préférés sont ceux pour lesquels l'une au moins, et de préférence toutes, les conditions ci-dessous sont respectées :
- R₁ et R₂ , pris ensemble, forment un cycle aromatique tel que décrit ci-dessus,
- R₃ est un hydrogène, un radical alkényle ayant de 2 à 6 atomes de carbone linéaire ou ramifié, un radical alkyle ayant de 1 à 6 atomes de carbone linéaire ou ramifié éventuellement substitué par un ou plusieurs atomes d'halogène ou un radical -OR₇
- R₄ est un hydrogène,
- R₅ est un radical de formule (i) ou (iii),
- R₆ est un hydrogène,
- R₈ est un radical OR₁₂,
- X et Y représentent CH,
- Z₁ est un atome d'oxygène ou de soufre.

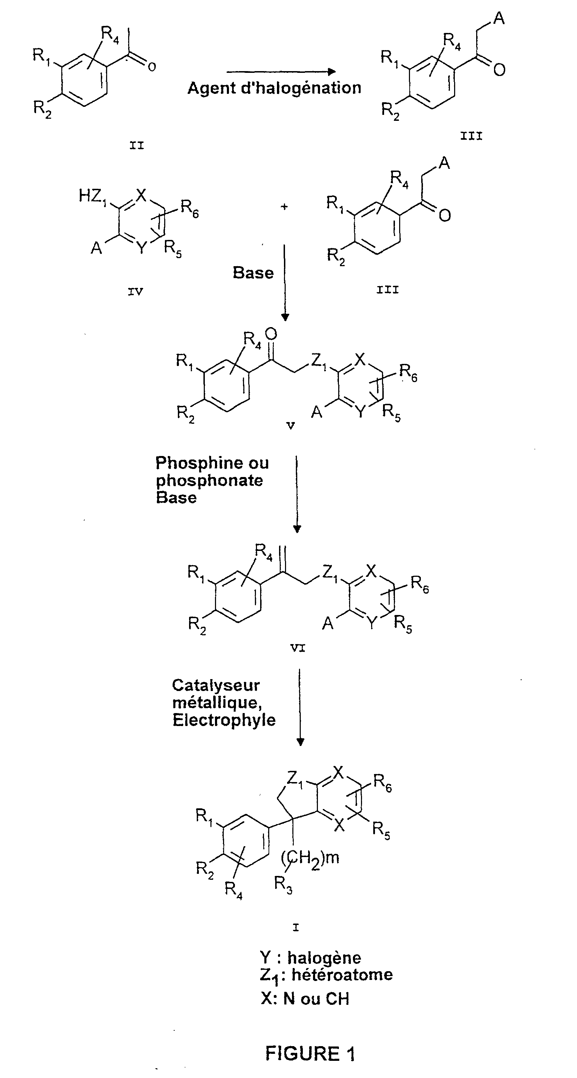

La présente invention a également pour objet les procédés de préparation des composés de formule (I), en particulier selon les schémas réactionnels donnés à la figure 1.

Ainsi les composés de formule générale (I) peuvent être obtenus (figure 1) à partir de la cétone (II), par halogénation, par exemple au moyen d'un agent de bromation tel que le brome. Le composé (III) obtenu est ensuite couplé avec le composé (IV), en présence de base telle que le carbonate de potassium ou l'hydrure de sodium. Le dérivé couplé (V) est soumis à l'action d'une phosphine ou d'un phosphonate en présence d'une base pour conduire au composé (VI). Le composé (VI) est cyclisé par action d'un catalyseur métallique comme le diacétate de palladium, en présence d'un donneur d'hydrure comme l'acide formique ou d'un nucléophile comme le vinyl tributyl étain ou l'acétate de lithium et si nécessaire d'une base. L'adjonction de sels ou de zéolites d'argent comme Ag₃PO₄ et de phosphines chirales comme le Binap permetd'obtenir un seul des énantiomères.

Les produits de formule générale (I) ainsi obtenus peuvent servir de produits de départ pour la fabrication d'autres composés de formule générale (I). Ces produits sont obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles sur le groupe R₅ comme indiqué ci-dessous:
acide carboxylique → ester
ester → acide carboxylique
acide → chlorure d' acide
chlorure d' acide → amide
acide → amide
acide → alcool
alcool → aldéhyde
amide → amine
thiol → thioéther
thioéther → sulfoxyde
thioéther → sulfone
acide sulfonique → ester sulfonique
acide sulfonique → sulfonamide
acide sulfinique → ester sulfinique

Lorsque R₃ représente le radical -COOH, les composés sont préférentiellement préparés en protégeant R₃ par un groupe protecteur de type allylique, benzylique ou tert-butylique.

Le passage à la forme libre peut être effectué :
- dans le cas d'un groupe protecteur allylique, au moyen d'un catalyseur tel certains complexes de métaux de transition en présence d'une amine secondaire.
- dans le cas d'un groupe protecteur benzylique, par débenzylation en présence d'hydrogène, au moyen d'un catalyseur tel le palladium sur charbon.
- dans le cas d'un groupe protecteur tert-butylique au moyen d'icdure de triméthylsilane.

Lorsque R₅ représente une fonction alcool les composés peuvent être obtenus à partir des dérivés aldéhydiques correspondants par action d'un hydrure alcalin, tel le borohydrure de sodium, dans un solvant alcoolique (par exemple le méthanol), ou par couplage du dérivé halogéné correspondant avec un dérivé de l'alcool 3-(tributylétain)allylique.

Lorsque R₅ représente une fonction aldéhyde, les composés peuvent être obtenus à partir des dérivés alcools par oxydation en présence d'oxyde de manganèse, de pyridinium dichromate ou du réactif de Swern.

Lorsque R₅ représente une fonction amide les composés peuvent être obtenus à partir des dérivés carboxyliques correspondants par réaction avec des amines aliphatiques, aromatiques, hétérocycliques soit par l'intermédiaire d'un chlorure d'acide ou en présence de dicyclohexylcarbodiimide ou de carbonyidiimidazole.

Certains de ces composés se lient aux récepteurs RXRs, les uns possédant une activité agoniste, les autres une activité antagoniste.

Les propriétes de binding et de transactivation comme agoniste aux récepteurs RXRs sont déterminées par des méthodes connues dans l'art, comme par exemple : MARTIN. B et all, Skin Pharmacol., 1992, **5**, 57-65 ; CAVEY. M. T. et al, Anal. Biochem., 1990, **186**, 19-23 ; LEVIN et al, Nature 1992, **355**, 359-61 ; ALLENBY et al, Proc. Natl. Acad. Sci., 1993, **90**, 30-4 ; ALLENBY et al, J. Biol. Chem., 1994, **269**, 16689-95.

L'activité agoniste RXRs est aussi déterminée par le test tel que décrit dans la demande de brevet français n° 95-07301 déposée le 19 juin 1995 par la demanderesse. Ce test comprend les étapes suivantes : (i) on applique topiquement sur une partie de la peau d'un mammifère une quantité suffisante d'un composé qui est un ligand actif d'au moins un récepteur de la superfamille des récepteurs nucléaires stéroïdiens/thyroïdiens autre qu'un ligand spécifique des récepteurs RXRs et pouvant s'hétérodimériser avec les RXRs tel qu'une molécule agoniste des RARs, (ii) on administre par voie systémique ou topique sur cette même partie de la peau du mammifère avant, pendant ou après l'étape (i) une molécule susceptible de présenter une activité agoniste des RXRs, (iii) on évalue la réponse sur la partie de la peau ainsi traitée du mammifère Ainsi la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des récepteurs RXRs.

L'activité antagoniste RXRα est évaluée dans le test de transactivation par détermination de la dose (IC₅₀) qui inhibe de 50% l'activité transactivatrice d'un agoniste sélectif RXRα: l'acide 6-(3,5,5,8,8-pentaméthyl-5,6,7,8-tétrahydro-2-naphthylthio)nicotinique (CD 2809) selon le protocole suivant :

Les cellules Hela sont co-transfectées avec un vecteur d'expression codant pour RXRα (p565-RXRα) et un plasmide rapporteur contenant l'élément de réponse 1/2 CRBP Il cloné en amont du promoteur hétérologue de la thymidine kinase et du gène rapporteur de la chloramphènicolm-acétyl-transfèrase (CAT). Dix-huit heures après co-transfection les cellules sont traitées avec une concentration fixe du CD 2809 et des concentrations croissantes de la molécule à évaluer. Après vingt-quatre heures de traitement le dosage de l'activité CAT est effectué par ELISA. La concentration fixe de CD2809 utilisée est 5 10⁻⁸M et correspond à son EC₅₀.

Certains des composés selon l'invention se lient aux récepteurs RARs et présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer research 38, p. 793-801, 1978). Ces tests montrent les activités de ces composés respectivement dans les domaines de la différenciation et de la prolifération cellulaire.

La présente invention a ainsi pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit: photo-induit ou chronologique, ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation, pour prévenir ou pour réparer les vergetures, ou encore pour favoriser la cicatrisation,
10) pour lutter contre les troubles de la fonction sébacée tels que l'hyperséborrhée de l'acné ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée, Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée.

Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### A. EXEMPLES DE COMPOSES

### EXEMPLE 1:

### 3-[3-(5,6,7,8-tetrahydro-5,5,8,8-tetraméthyl-2-naphtyl)-3-méthyl-2,3-dihydrobenzofuran-5-yl]-acrylate d'éthyle.

### (a) acide 3-iodo-4-hydroxybenzoique.

On additionne goutte à goutte une solution de perchlorate de sodium à 3,6% à un mélange d'acide 4-hydroxybenzoïque (12,75 g, 0,92 mol), de soude (3,7 g, 0,92 mol), d'iodure de sodium (13,85 g, 0,92 mol) dans le méthanol (350 ml), à 0°C. On laisse agiter deux heures à 0°C. On additionne 100 ml d'une solution de thiosulfate de sodium à 10%. Après agitation, on acidifie par de l'acide chlorhydrique jusqu'à pH 1. On extrait par 600 ml d'éther éthylique. La phase organique est lavée deux fois par 400 ml d' eau, séchée sur sulfate de magnésium et concentrée à l'évaporateur rotatif sous vide à 40°C.

Solide blanc. Masse: 28,76 g. Rendement : 100%.Pf: 157°C

RMN ¹H (DMSO, 250 MHz): 6,74 (1H Ar, d, J=8,4 Hz), 7,71 (1H Ar, d, J=8,4 Hz), 8,13 (1H Ar, s), 10,16 (1H, s), 11,12 (1H, s).

### (b) 3-iodo-4-hydroxybenzoate de méthyle.

On chauffe à reflux une solution d'acide 3-iodo-4-hydroxybenzoïque (28,76 g, 0,11 mol) et d'acide sulfurique (6,6 ml) dans le méthanol (160 ml), pendant 6 h. On ajoute 300 ml d'eau et on alcalinise avec du bicarbonate de soude jusqu'à neutralité. On extrait par de l'éther éthylique (600 ml). La phase organique est lavée deux fois avec 400 ml d'eau, séchée sur sulfate de magnésium, et concentrée à l'évaporateur rotatif sous vide à 40°C. Le produit est purifié par chromatographie flash sur colonne de silice (acétate d' éthyle 10 %, CH₂Cl₂ 90 %).

Solide blanc. Masse: 19,1 g, Rendement : 63%. Pf: 133°C

### (c) 4-[(5,6,7,8-tetrahydro-5,5,8,8-tetraméthyl-2-naphtoyl) méthyloxy]-3-iodobenzoate de méthyle.

Une solution de 5,6,7,8-tétrahydro-5,5,8,8-tétraméthyl-2-bromoacétonaphtone (9,8 g, 0,032 mol), de 4-hydroxy-3-iodobenzoate de méthyle (8,8 g, 0,032 mol),et de carbonate de potassium (8,5 g, 0,062 mol) dans la méthyléthylcétone (450 ml), est chauffée à reflux pendant 1 jour. On filtre, puis on concentre à l'évaporateur rotatif le milieu réactionnel. On ajoute 500 ml d'eau et 500 ml d'éther éthylique. Après agitation et décantation, la phase organique est lavée deux fois par 500 ml d'eau, séchée sur sulfate de magnésium et concentrée à l'évaporateur rotatif sous vide à 40 °C. Le produit est purifié par chromatographie flash sur colonne de silice (acétate d'éthyle 10 %, heptane 90 %).

Solide blanc. Masse: 9,56 g. Rendement : 60%. Pf. 125°C.

RMN ¹H (CDCL₃, 250 MHz): 1,30 (6H, s),1,32 (6H, s), 1,71 (4H, s), 3,88 (3H, s), 5,40 (2H, s), 6,70 (1H Ar, d, J=8,7Hz), 7,43 (1H Ar, d, J=8,5Hz), 7,74 (1H Ar, dd, J=2Hz, J=8,5 Hz), 7,93 (1H Ar, dd, J=8,7,J=2,3 Hz), 7,98 (1H Ar, d, J=2 Hz), 8,48 (1H Ar, d, J=2,3 Hz).

### (d) 3-iodo-4-[2-[5,6,7,8-tetrahydro-5,5,8,8-tetraméthyl-2-naphtyl)-1-propenylloxy benzoate de méthyle

On additionne en 8 heures à un mélange de 4-[(5,6,7,8-tétrahydro-5,5,8,8-tétraméthyl-2-naphtoyl) méthyloxy]-3-iodobenzoate de méthyle (7,50 g, 14,8 mmol) et de bromure de méthyltryphénylphoshine (7,30 g, 20,42 mmol) dans le THF (80 ml) une solution de méthylate de sodium à 30 % (2,67 g, 14,83 mmol).
On agite 18 h à température ambiante. Le mélange est concentré à l'évaporateur rotatif sous vide à 40°C. On extrait par 90 ml d'éther éthylique et 90 ml d'eau.

Après décantation, la phase organique est lavée deux fois par 90 ml d'eau, séchée sur sulfate de magnésium anhydre et concentrée à l'évaporateur rotatif sous vide à 40°C. Le produit est purifié par chromatographie flash sur colonne de silice (CH₂Cl₂ 70 %, heptane 30 %).

Solide blanc. Masse: 4,71 g, Rendement : 63%. Pf: 126°C

RMN ¹H (CDCL₃, 250 MHz): 1,29 (6H, s),1,30 (6H, s), 1,69 (4H, s), 3,89 (3H, s), 4,99 (2H, s), 5,55 (1H, s), 5,59 (1H, s), 6,87 (1H Ar, d, J=8,7Hz), 7,21 à 7,33 (2H Ar, m), 7,38 (1H Ar, d, J=1,8 Hz), 8,00 (1H Ar, dd, J=8,7,J=2 Hz), 8,48 (1H Ar, d, J=2 Hz).

RMN ¹³C (CDCL₃, 250 MHz): 31,79, 31,90, 34,16, 34,33, 34,96, 35,10, 52,09, 70,81, 85,85, 111,35, 112,73, 114,05, 123,33, 124,17, 124,46, 126,71, 129,67, 131,45, 131,74, 135,23, 141,06, 141,99, 145,05, 145,10, 160,67, 165,47.

### (e) 3-(5,6,7,8-tetrahydro-5,5,8,8-tetraméthyl-2-naphtyl)-(3-méthyl)-2,3-dihydro-benzofuran-5-carboxylate de méthyle.

On chauffe à 95°C pendant 4 h un mélange de tributhylamine (2,28 ml, 9,6 mmol), de diacétate de palladium (0,06 g, 0,3 mmol), d'acide formique (0,29 ml, 7,4 mmol) et de 3-lodo-4-[2-[5,6,7,8-tétrahydro-5,5,8,8-tétraméthyl-2-naphtyl]-1-propénylloxy benzoate de méthyle (1,37 g, 2,72 mmol) dans l'acétonitrile (25 ml). Le milieu réactionnel est concentré à l'évaporateur rotatif sous vide à 40°C, On ajoute 40 ml d'eau et 40 ml d'éther d'éthylique. Après décantation, la phase organique est lavée deux fois par 20 ml d'eau, séchée sur sulfate de magnésium, et concentrée à l'évaporateur rotatif sous vide à;40°C. Le produit est purifié par chromatographie flash sur colonne de silice (CH₂Cl₂ 50% heptane 50 %)

Solide blanc. Masse: 630 mg, Rendement: 61%. Pf: 74°C

RMN ¹H (CDCL₃, 250 MHz): 1,20 à 1,24 (12H, m), 1,65 (4H, s), 1,73 (3H, s), 3,83 (3H, s), 4,51 (1H, d, J=8,7 Hz), 4,66 (1H, d, J=8,7 Hz), 6,87 (1H Ar, d, J=8,3Hz), 6,96 (1H Ar, dd, J=8,3,J=2 Hz), 7,19 à 7,24 (2H Ar, m), 7,73 (1H Ar, d, J=1,8 Hz), 7,92 (1H Ar, dd, J=8,3,J=2 Hz).

RMN ¹³C (CDCL₃, 250 MHz): 26,63, 31,99, 32,08, 32,11, 34,18, 35,21, 35,32, 49,45, 52,04, 87,37, 109,82, 123,31, 123,98, 124,36, 126,46, 126,85, 131,41, 136,62, 142,56, 143,49, 145,04, 163,91, 167,19.

### (f) acide 3-(5,6,7,8-tetrahydro-5,5,8,8-tetraméthyl-2-naphtyl)-3-méthyl-2,3-dihydro-benzofuran-5-carboxylique

On agite 5 jours à température ambiante un mélange de 3-(5,6,7,8-tétrahydro-5,5,8,8-tétraméthyl-2-naphtyl)-(3-méthyl)-2,3-dihydro-benzofuran-5-carboxylate de méthyle (510 mg, 1,35 mmol), de soude (0,33 g, 7,9 mmol), et d'hydroxyde de lithium (0,33 g, 7,9 mmol). On concentre à l'évaporateur rotatif sous vide à 40°C.

On rajoute 10 ml d'eau et 10 ml d'acétate d'éthyle. On acidifie le mélange par une solution d'acide chlorhydrique concentrée jusqu'à pH 1. Après décantation, la phase organique est lavée deux fois par 10 ml d' eau, séchée sur sulfate de magnésium, et concentrée à l'évaporateur rotatif sous vide à 40°C. Le solide obtenu est lavé à l'heptane.

Solide blanc. Masse: 400 mg, Rendement : 83%. Pf: 246°C

RMN ¹H (DMSO, 250 MHz): 1,20 à 1,23 (12H, m), 1,64 (4H, s), 1,74 (3H, s), 3,83 (3H, s), 4,44 (1H, d, J=8,7 Hz), 4,66 (1H, d, J=8,7 Hz), 6,85 (1H Ar, d, J=7,5Hz), 6,96 (1H Ar, dd, J=8,3,J=2 Hz), 7,19 à 7,24 (2H Ar, m), 7,73 (1H Ar, d, J=1,8 Hz), 7,92 (1H Ar, dd, J=8,3,J=2 Hz).

RMN ¹³C (DMSO, 250 MHz): 26,26, 31,63, 31,72, 31,75, 33,83, 34,25, 34,96, 35,06, 49,14, 87,01, 109,34, 123,36, 123,74, 124,03, 126,41, 126,52, 131,38, 136,25, 142,39, 143,02, 144,60, 163,59, 167,90.

### (g) 3-(5,6,7,8-tetrahydro-5,5,8,8-tetraméthyl-2-naphthyl)-3-méthyl-2,3-dihydro-benzofuran-5-méthanol

On additionne goutte à goutte à 0°C 7,7 ml d'une solution 1M de borane dans le THF, à une solution d'acide 3-(5,6,7,8-tétrahydro-5,5,8,8-tétraméthyl-2-naphtyl)-3-méthyl-2,3-dihydro-benzofuran-5-carboxylique (1,7g, 4,7mol) dans le THF (10 ml). On agite 4 heures à température ambiante puis 2 ml d'une solution de THF et d'eau (1:1) sont additionnés. Après concentration à l'évaporateur rotatif sous vide à 40°C. On extrait à l'acétate d'éthyle. La phase organique est lavée à l'eau, séchée sur sulfate de magnésium anhydre, concentrée à l'évaporateur rotatif sous vide à 40°C. Le produit est purifié par chromatographie flash sur colonne de silice.

Amorphe jaune. Masse: 1,7 g, Rendement : 100%.

RMN δ ppm :
¹H (CDCl₃): 1,20 à 1,25 (12H, m), 1,66 (4H, s), 1,72 (3H, s), 3,47 (1H, s), 4,44 (1H, d, J=8,8 Hz), 4,59 (2H, s), 4,60 (1H, d, J=8,8 Hz), 6,84 (1H Ar, d, J=8Hz), 7,01 (1H Ar, dd, J=8,3,J=2,3 Hz), 7,05 (1H Ar, d, J=1,8Hz), 7,17 à 7,22 (3H Ar, m).
¹³C (CDCl₃): 25,86, 31,39, 31,48, 31,51, 33,55, 33,97, 34,65, 34,75, 49,25, 65,05, 86,02, 109,29, 123,07, 123,45, 123,78, 126,11, 127,26, 133,18, 135,93, 142,38, 142,65, 144,27, 159,00.

### (h) 3-(5,6,7,8-tetrahydro-5,5,8,8-tetraméthyl-2-naphtyl)-3-méthyl-2,3-dihydro-benzofuran-5-carbaldéhyde.

Un mélange d'alcool précedement obtenu (1g, 2,86 mmol), de dichromate de pyridinium 2,15g, 5,7 mmol) dans le dichlorométhane est agité à température ambiante 3h. Après filtration et concentration à l'évaporateur rotatif sous vide à 40°C, le produit est purifié par chromatographie flash sur colonne de silice.

Huile. Masse: 0,98 g, Rendement: 98%.

RMN δ ppm:
¹H (CDCl₃): 1,20 à 1,26 (12H, m), 1,67 (4H, s), 1,76 (3H, s), 4,57 (d,1H, J=8,9), 4,73 (d,1H, J=8,9), 6.96 (1H Ar, s), 7,00 (1H Ar, s), 7,20 à 7,25 (2H Ar, m), 7,59 (1H Ar, d, J=1,5Hz), 7,74 (1H Ar, dd, J=8,3 Hz, J=1,8Hz), 9.83 (1H, s).
¹³C (CDCl₃): 26,44, 31,77, 31,87, 31,91, 33,99, 34,39, 34,98, 35,09, 49,04, 87,45, 110,26, 123,74, 124,12, 125,52, 126,75, 130,90, 132,95, 137,68, 141,99, 143,48, 144,94, 155,10, 190,67.

### (i) 3-[3-(5,6,7,8-tetrahydro-5,5,8,8-tetraméthyl-2-naphtyl)-3-méthyl-2,3-dihydro-benzofuran-5-yl]-acrylate d'éthyle

De l'hydrure de sodium à 80% dans l'huile (41 mg, 1,38 mmol), est additionné à un mélange d'aldéhyde précedement obtenu et de triéthylphosphonoacétate (0,27 ml, 1,38 mmol) dans le THF (10ml). Le mélange est agité 4h à température ambiante, extrait à l'acétate d'éthyle et lavé à l'eau. Après séchage la phase organique est concentré à l'évaporateur rotatif sous vide à 40°C, le produit est purifié par chromatographie flash sur colonne de silice.

Huile. Masse: 330 mg, Rendement : 69%.

RMN δ ppm:
¹H (CDCl₃): 1,18 à 1,33 (15H, m), 1,67 (4H, s), 1,73 (3H, s), 4,22 (2H, q, J=7,1Hz), 4,49 (1H, d, J=8,8Hz), 4,63 (1H, d, J=8,8Hz), 6,24 (1H, d, 15,8Hz), 6,87 (1H Ar, d, J=8,5Hz), 6,99 (1H Ar, dd, J=8,3Hz, J=2,3Hz), 7,21 à 7,24 (3H Ar, m), 7,36 (1H Ar, d, J=8,3Hz), 7,62 (1H Ar, d, J=15,8Hz).
¹³C (CDCl₃): 14,36, 26,34, 31,79, 31,91, 33,99, 34,40, 35,03, 35,14, 49,47, 60,25, 86,93, 110,23, 115,24, 123,79, 124,21, 126,66, 127,83, 129,67, 137,10, 142,32, 143,32, 144,71, 144,86, 161,79, 167,38.

### EXEMPLE 2:

### Acide 3-[3-(5,6,7,8-tetrahydro-5,5,8,8-tetraméthyl-2-naphtyl)-3-méthyl-2,3-dihydro-benzofuran-5-yl]-acrylique

### (a) acide 3-[3-(5,6,7,8-tetrahydro-5,5,8,8-tetraméthyl-2-naphtyl)-3-méthyl-2,3-dihydro-benzofuran-5-yl]-acrylique

Un mélange de 3-[3-(5,6,7,8-tetrahydro-5,5,8,8-tetraméthyl-2-naphtyl)-3-méthyl-2,3-dihydro-benzofuran-5-yl]-acrylate de méthyle (330mg, 0,79 mmol), d'une solution de soude méthanolique 2N (4ml, 7,9 mmol) dans le THF (3ml) est chauffée 5heures à 60°C. Le mélange est acidifié à pH 1 par une solution d'acide chlorhydrique concentré, extrait à l'acétate d'éthyle et lavé à l'eau. Après séchage la phase organique est concentré à l'évaporateur rotatif sous vide à 40°C, le produit est purifié par chromatographie flash sur colonne de silice.

Solide jaune. Masse : 45 mg, Rendement : 58%. Pf: 160°C.

RMN δ ppm:
¹H (CDCl₃): 1,21 à 1,26 (12H, m), 1,67 (4H, s), 1,74 (3H, s), 4,50 (2H, q, J=7Hz), 4,64 (1H, d, J=8,8Hz), 4,63 (1H, d, J=8,8Hz), 6,27 (1H, d, 15,8Hz), 6,88 (1H Ar, d, J=8,5Hz), 6,99 (1H Ar, d, J=8,3Hz), 7,21 à 7,24 (3H Ar, m), 7,38 (1H Ar, d, J=8,3Hz), 7,69 (1H Ar, d, J=15,8Hz).
¹³C (CDCl₃): 26,35, 31,78, 31,89, 33,96, 34,38, 35,02, 35,11, 49,43, 86,93, 110,27, 115,05, 123,74, 124,06, 124,16, 126,65, 127,68, 129,92, 137,11, 142,26, 143,31, 144,86, 146,24, 161,98, 172,16.

### EXEMPLE 3:

### Acide [3-méthyl-3-(5,5,8,8-tetraméthy)-5,6,7,8-tetrahydro-naphtalen-2-yl)-2,3-dihydro-benzofuran-5-yl]-propynoique.

### a) 5-(2,2dibromovinyl)-3-méthyl-3-(5,5,8,8-tetraméthyl-5,6,7,8-tetrahydro-naphtalen-2-yl)-2,3-dihydro-benzofurane

Du tétrabromométhane (1,22 g, 3,67 mmol) est additionné à un mélange de triphénylphosphine (1,93 g, 7,35 mmol) dans le dichlorométhane (10 ml), à 0°C.

Le mélange est agité à température ambiante 1h, puis une solution de 3-(5,6,7,8-tetrahydro-5,5,8,8-tetraméthyl-2-naphtyl)-3-méthyl-2,3-dihydro-benzofuran-5-carbaldéhyde (850 mg, 2,45 mmol) dans le dichiorométhane (2 ml) est additionnée à 0°C. L'agitation est poursuivie 2h à température ambiante, puis la suspension est concentrée à l'évaporateur rotatif sous vide. Le produit est purifié par chromatographie sur colonne de silice.

Huile incolore. Masse: 860 mg, Rendement: 70 %.

### b) Acide [3-méthyl-3-(5,5,8,8-tetraméthyl-5,6,7,8-tetrahydro-naphtalen-2-yl)-2,3-dihydro-benzofuran-5-yl]-propynoique

Une solution de butyllithium 1,6M (2,35 ml, 3,55 mmol) est additionnée à une solution de 5-(2,2-dibromovinyl)-3-Méthyl-3-(5,5,8,8-tetraméthyl-5,6,7,8-tetrahydro-naphtalen-2-yl)-2,3-dihydro-benzofurane (860 mg, 1,71 mmol) dans le THF (20ml) à -78°C. L'agitation est poursuivie 1h à -78°C puis on fait buller du dioxide de carbone pendant 15mn. La température est laissée remonter à température ambiante. La solution est traitée par de l'acétate d'éthyle et par une solution de chlorure d'ammonium, la phase organique est lavée deux fois à l'eau, séchée sur sulfate de magnésium, et concentrée à l'évaporateur rotatif sous vide à 40°C. Le solide obtenu est recristallisé dans un mélange heptane/éther éthylique.

Solide blanc. Masse: 98 mg. F= 125°C

RMN ¹H (CDCL₃, 250 MHz): 1,22 à 1,25 (12H, d), 1,66 (4H, s), 1,71 (3H, s), 4,50 (1H, d, J=8,7 Hz), 4,67 (1H, d, J=8,7 Hz), 6,64 (1H Ar, d, J=8,5Hz), 6,94 (1H Ar, dd), 7,13 à 7,24 (3H Ar, m), 7,45 (1H Ar, dd).

### EXEMPLE 4:

### (+)-3-[3-méthyl-3-(5,5,8,8-tetraméthyl-5,6,7,8-tetrahydro-naphtalen-2-yl)-2,3-dihydro-benzofuran-5-yl]-acrylate d'éthyle

### a) (+)-3-[3-méthyl-3-(5,5,8,8-tetraméthyl-5,6,7,8-tetrahydro-naphtalen-2-yl)-2,3-dihydro-benzofuran-5-yl]-carboxylate de méthyle.

On chauffe à 60°C pendant 4j un mélange de carbonate de calcium (100 mg, 1 mmol), de diacétate de palladium (10 mg, 0,05 mmol), de formiate de sodium (68 mg, 1 mmol), de 3-lodo-4-[2-[5,6,7,8-tétrahydro-5,5,8,8-tétraméthyl-2-naphtyl]-1-propényloxy benzoate de méthyle (250 mg, 05 mmol), de (S)-(-)-2,2'-Bis(diphenylphosphino)-1,1'-binaphtyl (65 mg, 0,1 mmol), et de zeolite d'argent (Aldrich 36,660-9) dans l'acétonitrile (7 ml). Le milieu réactionnel est filtré sur célite, concentré à l'évaporateur rotatif sous vide à 40°C. On ajoute de l'eau et de l'éther d'éthylique. Après décantation, la phase organique est lavée deux fois à l'eau, séchée sur sulfate de magnésium, et concentrée à l'évaporateur rotatif sous vide à 40°C. Le produit est purifié par chromatographie flash sur colonne de silice.

Solide blanc. Masse: 75 mg, Rendement: 40%. α_{d}[CHCl₃]: +116.

La suite de la synthèse est identique à celle du mélange racémique (exemple 1).

### b) (+)-3-[3-méthyl-3-(5,5,8,8-tetraméthyl-5,6,7,8-tetrahydro-naphtalen-2-yl)-2,3-dihydro-benzofuran-5-yl]-acrylate d'éthyle

α_{d}[CHCl₃]: +227.

### EXEMPLE 5:

### Acide (+)-3-[3-méthyl-3-(5,5,8,8-tetraméthyl-5,6,7,8-tetrahydro-naphtalen-2-yl)-2,3-dihydro-benzofuran-5-yl]-acrylique

α_{d}[CHCl₃]: +290.

### EXEMPLE 6:

### (-)-3-[3-méthyl-3-(5,5,8,8-tetraméthyl-5,6,7,8-tetrahydro-naphtalen-2-yl)-2,3-dihydro-benzofuran-5-yl]-acrylate d'éthyle

### a) (-)-3-[3-Méthyl-3-(5,5,8,8-tetraméthyl-5,6,7,8-tetrahydro-naphtalen-2-yl)-2,3-dihydro-benzofuran-5-yl]-carboxylate de méthyle.

Le protocole opératoire est le même que celui suivi dans l'exemple 4a en employant le (R)-(+)-2,2'-Bis(diphenylphosphino)-1,1'-binaphtyl comme ligand du diacétate de palladium.

α_{d}[CHCl₃]: -145.

La suite de la synthèse est identique à celle du mélange racémique (exemple 1).

### b) (-)-3-[3-méthyl-3-(5,5,8,8-tetraméthyl-5,6,7,8-tetrahydro-naphtalen-2-yl)-2,3-dihydro-benzofuran-5-yl]-acrylate d'éthyle.

α_{d}[CHCl₃]: -238

### EXEMPLE 7:

### Acide (-)-3-[3-méthyl-3-(5,5,8,8-tetraméthyl-5,6,7,8-tetrahydro-naphtalen-2-yl)-2,3-dihydro-benzofuran-5-yl]-acrylique.

α_{d}[CHCl₃]: -306.

### EXEMPLE 8:

### 3-[3-méthyl-3-(3,5,5,8,8-pentaméthyl-5,6,7,8-tetrahydro-naphtalen-2-yl)-2,3-dihydro-benzofuran-5-yl]-acrylate d'éthyle

### a) 3-lodo-4-[2-oxo-2-(3,5,5,8,8-pentaméthyl-5,6,7,8-tetrahydro-naphtalen-2-yl)-ethoxy]-benzoate de méthyle.

Le protocole expérimental est analogue à celui suivi pour l'exemple 1c appliqué au 3-iodo-4-hydroxybenzoate de méthyle et à la 5,6,7,8-tétrahydro 3,5,5,8,8-pentaméthyl-2-bromoacetonaphtone.
Poudre blanche. Rendement: 1.65 g (86%). F=89°C. ¹H NMR (CDCl₃) d: 1.29 (6H, s),1.31 (6H, s), 1.70 (4H, s), 2.49 (3H, s), 3.88 (3H, s), 5.30 (2H, s), 7.19 (1H Ar, s), 7.30 (1H Ar, s), 7.37 (1H Ar, d, J=8 Hz), 7.63 (1H Ar, s), 7.87 (1H Ar, d, J=8 Hz).

### b) 3-lodo-4-[2-(3,5,5,8,8-pentaméthyl-5,6,7,8-tetrahydro-naphtalen-2-yl)-allyloxy]-benzoate de méthyle.

Le protocole expérimental est analogue à celui suivi pour l'exemple 1d appliqué au 3-lodo-4-[2-oxo-2-(3,5,5,8,8-pentaméthyl-5,6,7,8-tetrahydro-naphtalen-2-yl)-ethoxy]-benzoate de méthyle.

Huile incolore. Masse: 3.2 g. Rendement: 40%.

¹H NMR (CDCl₃) d: 1.26 (6H, s), 1.29 (6H, s), 1.67 (4H, s), 2.29 (3H, s), 3.89 (3H, s), 4.75 (2H, s), 5.23 (1H, d, J=1.6 Hz), 5.77 (1H, d, J=1.6 Hz), 6.79 (1H Ar, d, J=8.7Hz), 7.09 (1H Ar, s), 7.13 (1H Ar, s), 7.98 (1H Ar, dd, J=8.7 Hz, J=2.1 Hz), 8.47 (1H Ar, d, J=2.1 Hz).

### c) 3-(5,6,7,8-tetrahydro-3,5,5,8,8-pentaméthyl-2-naphtyl)-3-méthyl-2,3-dihydro-benzofuran-5-carboxylate de méthyle.

Le protocole expérimental est analogue à celui suivi pour l'exemple 1e appliqué au 3-iodo-4-[2-(3,5,5,8,8-pentaméthyl-5,6,7,8-tetrahydro-naphtalen-2-yl)-allyloxy]-benzoate de méthyle.

Huile incolore. Masse: 1 g. Rendement: 42%.

¹H NMR (CDCl₃) d: 1.24 à 1.28 (12H, m), 1.67 (4H, s), 1.76 (3H, s), 1.92 (3H, s), 3.85 (3H, s), 4.52 (1H, d, J=9,1 Hz), 4.81 (1H, d, J=9.1 Hz), 6.85 (1H Ar, d, J=8.4 Hz), 7.01 (1H Ar, s), 7.32 (1H Ar, s), 7.65 (1H Ar, d, J=1.8 Hz), 7.92 (1H Ar, dd, J=8.4 Hz, J=1.8 Hz).

¹³C NMR (CDCl₃) d: 21.15 (CH₃), 29.52 (CH₃), 31.61 (CH₃ TTNN), 31.71 (CH₃ TTNN), 31.88 (CH₃ TTNN), 31.95 (CH₃ TTNN), 33.72 (C TTNN), 34.07 (C TTNN), 35.14 (CH₂ TTNN), 35.22 (CH₂ TTNN), 49.28 (C), 51.82 (OCH₃), 85.32 (CH₂O), 109.30 (CH Ar), 122.78 (C Ar), 125.14 (CH Ar), 124.36 (CH Ar), 125.96 (CH Ar), 130.77 (CH Ar), 131.12 (CH Ar), 131.56 (C Ar), 133.53 (C Ar), 138.60 (C Ar), 141.86 (C Ar), 143.64 (C Ar), 163.67 (C-O Ar), 167.29 (COO).

### d) 3-(5,6,7,8-tetrahydro-3,5,5,8,8-pentaméthyl-2-naphtyl)-3-méthyl-2,3-dihydro-benzofuran-5-carbaldéhyde.

Une solution 1M d'hydrure de diisobutylaluminium dans le toluène (5,82 ml, 5,82 mmol) est additionnée à 0°C, goutte à goutte à une solution de 3-(5,6,7,8-tetrahydro-3,5,5,8,8-pentaméthyl-2-naphtyl)-3-méthyl-2,3-dihydro-benzofuran-5-carboxylate de méthyle (1 g, 2,55 mmol) dans le toluène (30 ml). La solution est agitée 1h à 0°C, puis traitée par une solution de tartrate double de sodium et potassium, filtrée sur celite et reprise dans un mélange d'éther éthylique et d'eau.

La phase organique est lavée à l'eau, séchée sur sulfate de magnésium et concentrée à l'évaporateur rotatif sous vide à 40°C.

Un mélange d'alcool précedement obtenu (1g, 2,55 mmol), de dichromate de pyridinium (2 g, 5,3 mmol) dans le dichlorométhane est agité à température ambiante 4h. Après filtration et concentration à l'évaporateur rotatif sous vide à 40°C, le produit est purifié par chromatographie flash sur colonne de silice.

Huile. Masse: 470 mg, Rendement: 51%.

¹H NMR (CDCl₃) d: 1.25 à 1.27 (12H, m), 1.67 (4H, s), 1.78 (3H, s), 1.91 (3H, s), 4.56 (1H, d, J=9.2 Hz), 4.85 (1H, d, J=9.2 Hz), 6.95 (1H Ar, d, J=8.2 Hz), 7.02 (1H Ar, s), 7.32 (1H Ar, s), 7.52 (1H Ar, d, J=1.7 Hz), 7.72 (1H Ar, dd, J=8.2 Hz, J=1.7 Hz), 9,82 (1H, s).

### e) 3-[3-méthyl-3-(3,5,5,8,8-pentaméthyl-5,6,7,8-tetrahydro-naphtalen-2-yl)-2,3-dihydro-benzofuran-5-yl]-acrylate d'éthyle

Le protocole expérimental est analogue à celui suivi pour l'exemple 1i appliqué au 3-(5,6,7,8-tetrahydro-3,5,5,8,8-pentaméthyl-2-naphtyl)-3-méthyl-2,3-dihydro-benzofuran-5-carbaldehyde.

Huile incolore. Masse: 320 mg. Rendement: 79%.

¹H NMR (CDCl₃) d: 1.25 à 1.33 (15H, m), 1.68 (4H, s), 1.76 (3H, s), 1.94 (3H, s), 4.21 (2H, q, J=7.2 Hz), 4.49 (1H, d, J=9.1 Hz), 4.78 (1H, d, J=9.1 Hz), 6.23 (1H, d, J=16 Hz), 6.83 (1H Ar, d, J=8.3 Hz), 7.02 (1H Ar, s), 7.15 (1H Ar, s), 7.31 (1H Ar, d, J=1.8 Hz), 7.34 (1H Ar, d, J=8.3 Hz), 7.61 (1H, d, J=16 Hz),

### EXEMPLE 9:

### Acide 3-[3-méthyl-3-(3,5,5,8,8-pentaméthyl-5,6,7,8-tetrahydro-naphtalen-2-yl)-2,3-dihydro-benzofuran-5-yl]-acrylique.

Une solution de 3-[3-Méthyl-3-(3,5,5,8,8-pentaméthyl-5,6,7,8-tetrahydro-naphtalen-2-yl)-2,3-dihydro-benzofuran-5-yl]-acrylate d'éthyle (320 mg, 0,74 mmol), d'eau (40 ul) et d' hydroxyde de sodium (240 mg, 6 mmol) dans le THF est chauffée à reflux 24h. Le mélange réactionnel est versé sur un mélange AcOEt/eau, acidifié à pH=1 par une solution d'acide chlorhydrique concentrée, et extrait une fois à l'acétate d'éthyle. Après décantation la phase organique est lavée deux fois à l'eau, séchée sur sulfate de manésium et concentrée à l'évaporateur rotatif sous vide à 40°C. Le solide obtenu est cristallisé dans l'heptane.

Solide blanc.Masse: 300 mg. Rendement: 100%. Pf: 190°C.

¹H NMR (CDCl₃): 1.24 à 1.27 (12H, m), 1.68 (4H, s), 1.77 (3H, s), 1.95 (3H, s), 4.50 (1H, d, J=9.1 Hz), 4.79 (1H, d, J=9.1 Hz), 6.22 (1H, d, J=15,9 Hz), 6.85 (1H Ar, d, J=8.3 Hz); 7.03 (1H Ar, s), 7.17 (1H Ar, s), 7.31 (1H Ar, s), 7.37 (1H Ar, d, J=8.3 Hz), 7.70 (1H, d, J=15.9 Hz).

¹³C NMR (CDCl₃):21,1, 29,2, 31,5, 31,7, 31,8, 31,9, 33,6, 33,9, 35,0, 35,1, 49,4, 84,8, 109,8, 115,7, 123,4, 125,0, 127,4, 129,5, 130,6, 133,1, 137,1, 138,7, 141,7, 143,4, 144,5, 161,4, 168,9, 186,4.

### EXEMPLE 10:

### 3-[3-méthyl-3-(naphtalen-2-yl)-2,3-dihydro-benzofuran-5-yl]-acrylate d'éthyle

### a) 3-lodo-4-[2-(naphtalen-2-yl)-2-oxo-ethoxy]-benzoate de méthyle.

Le protocole expérimental est analogue à celui suivi pour l'exemple 1c appliqué à la 2-Bromo-1-naphtalen-2-yl-éthanone et au 4-hydroxy-3-iodobenzoate de méthyle.

Solide beige. Masse: 14,7 g. Rendement: 91%.

¹H NMR (CDCl₃): 3,88 (3H, s), 5,51 (2H, s), 6,77 (1H, d, J=8,7 Hz), 7,58 à 8,05 (8H Ar, m), 8,48 (1H Ar, d, J=2,05 Hz).

### b) 3-lodo-4-[2-(naphtalen-2-yl)-allyloxy]-benzoate de méthyle

Le protocole expérimental est analogue à celui suivi pour l'exemple 1d appliqué au 3-lodo-4-[2-(naphtalen-2-yl)-2-oxo-ethoxy)-benzoate de méthyle.

Solide jaune. Masse: 2,9 g. Rendement: 20%.

¹H NMR (CDCl₃): 3,88 (3H, s), 5,12 (2H, s), 5,71 (1H, s), 5,80 (1H, s), 6,92 (1H, d, J=8,7 Hz), 7,48 à 7,52 (2H Ar, m), 7,63 (1H Ar, dd, J=1,8 Hz, 8,7 Hz), 7,852 à 7,99 (3H Ar, m), 8,01 (1H Ar, dd, J=8,7 Hz), 8,48 (1H Ar, d, J=2,2 Hz).

### c) 3-méthyl-3-naphtalen-2-yl-2,3-dihydro-benzofuran-5-carboxylate de méthyle.

Le protocole expérimental est analogue à celui suivi pour l'exemple 1e appliqué au 3-lodo-4-[2-(naphtalen-2-yl)-allyloxy]-benzoate de méthyle. Huile incolore. Masse: 1 g. Rendement: 48%.

¹H NMR (CDCl₃): 1,89 (3H, s), 3,88 (3H, s), 4,62 (1H, d, J=8,9 Hz), 4,80 (1H, d, J=8,9 Hz), 6,93 (1H, d, J=8,5 Hz), 7,34 (1H, dd, J=2 Hz, J=8,7 Hz), 7,45 à 7,49 (2H, m), 7,71 à 7,73 (2H, m), 7,78 à 7,82 (3H, m), 7,97 (1H, dd, J=1,8 Hz, J=8,5 Hz).

### d) 3-méthyl-3-naphtalen-2-yl-2,3-dihydro-benzofuran-5-carbaldéhyde

Le protocole expérimental est analogue à celui suivi pour l'exemple 8d appliqué au 3-méthyl-3-naphtalen-2-yl-2,3-dihydro-benzofuran-5-carboxylate de méthyle.

Huile incolore. Masse: 770 mg. Rendement: 85%.

¹H NMR (CDCl₃): 1,91 (3H, s), 4,67 (1H, d, J=9 Hz), 4,85 (1H, d, J=9 Hz), 7,02 (1H, d, J=8,3 Hz), 7,36 (1H, dd, J=2 Hz, J=8,7 Hz), 7,46 à 7,50 (3H, m), 7,60 (1H, d, J=1,7 Hz), 7,74 à 7,82 (4H, m), 8,82 (1H, s).

### e) 3-[3-méthyl-3-(naphtalen-2-yl)-2,3-dihydro-benzofuran-5-yl]-acrylate d'éthyle

Le protocole expérimental est analogue à celui suivi pour l'exemple 1i appliqué au 3-méthyl-3-(naphtalen-2-yl)-2,3-dihydro-benzofuran-5-carbaldehyde.

Huile incolore. Masse: 320 mg. Rendement: 79%.

¹H NMR (CDCl₃): 1,28 (3H, t, J=7 Hz), 1,88 (3H, s), 4,20 (2H, q, J=7 Hz), 4,59 (1H, d, J=8,9 Hz), 4,76 (1H, d, J=8,9 Hz), 6,23 (1H, d, J=16 Hz), 6,92 (1H, d, J=8,3 Hz), 7,21 (1H, d, J=1,7 Hz), 7,35 à 7,49 (4H, m), 7,61 (1H, d, J=15,9 Hz), 7,74 à 7,82 (4H, m).

### EXEMPLE 11:

### Acide 3-[3-méthyl-3-(naphtalen-2-yl)-2,3-dihydro-benzofuran-5-yl]-acrylique.

Le protocole expérimental est analogue à celui suivi pour l'exemple 9 appliqué au 3-[3-méthyl-3-(naphtalen-2-yl)-2,3-dihydro-benzofuran-5-yl]-acrylate d'éthyle.

Huile incolore. Masse: 650 mg. Rendement: 97%. F=160°C.

¹H NMR (CDCl₃): 1,88 (3H, s), 4,59 (1H, d, J=8,9 Hz), 4,76 (1H, d, J=8,9 Hz), 6,21 (1H, d, J=15,9 Hz), 6,92 (1H, d, J=8,3 Hz), 7,22 (1H, s), 7,33 à 7,93 (9H, m).

**EXEMPLE 12:**

### 3-[3-(8,8-diméthvl-5,6,7,8-tetrahydro-naphtalen-2-yl)-3-méthyl-2,3-dihydro-benzofuran-5-yl]-acrylate d'éthyle

### a) 4-[2-(8,8-diméthyl-5,6,7,8-tetrahydro-naphtalen-2-yl)-2-oxo-ethoxy]-3-iodo-benzoate de méthyle.

Le protocole expérimental est analogue à celui suivi pour l'exemple 1c appliqué à la 5,6,7,8-tétrahydro-8,8-diméthyl-2-bromoacétonaphtone et au 4-hydroxy-3-iodobenzoate de méthyle.

Solide beige. Masse: 18,7 g. Rendement: 64%. F=120°C

¹H NMR (CDCl₃): 1.31 (6H, s), 1.66 à 1.71 (2H, m), 1.79 à 1.89 (2H, m), 2.82 (2H, t, J=6.1 Hz), 3.88 (3H, s), 5.39 (2H, s), 6.71 (1H Ar, d, J=8.8 Hz), 7.16 (1H Ar, d, J=8.0 Hz), 7.69 (1H Ar, dd, J=1.7 Hz, J=8.0 Hz), 7.93 (1H Ar, dd, J=8.8 Hz,J=2.0 Hz), 7.99 (1H Ar, d, J=1.7 Hz), 8.47 (1H Ar, d, J=2.0 Hz).

### b)4-[2-(8,8-diméthyl-5,6,7,8-tetrahydro-naphtalen-2-yl)-allyloxy]-3-iodo-benzoate de méthyle.

Le protocole expérimental est analogue à celui suivi pour l'exemple 1d appliqué au 4-[2-(8,8-diméthyl-5,6,7,8-tetrahydro-naphtalen-2-yl)-2-oxo-ethoxy]-3-iodo-benzoate de méthyle.

Huile incolore. Masse: 11 g. Rendement: 59%.

¹H NMR (CDCl₃): 1.30 (6H, s), 1.65 à 1.69 (2H, m), 1.77 à 1.86 (2H, m), 2.77 (2H, t, J=6.1 Hz), 3.89 (3H, s), 4.99 (2H, s), 5.55 (1H, s), 5.57 (1H, s), 6.87 (1H Ar, d, J=8.6 Hz), 7.05 (1H Ar, d, J=7.9 Hz), 7.17 (1H Ar, dd, J=7.9 Hz, J=1.8 Hz), 7.40 (1H Ar, d, J=1.8 Hz), 7.99 (1H Ar, dd, J=8.6 Hz, J=2.1 Hz), 8.47 (1H Ar, d, J=2.1 Hz).

### c) 3-(8,8-diméthyl-5,6,7,8-tetrahydro-naphtalen-2-yl)-3-méthyl-2,3-dihydro-benzofuran-5-carboxylate de méthyle.

Le protocole expérimental est analogue à celui suivi pour l'exemple 1e appliqué au 4-[2-(8,8-diméthyl-5,6,7,8-tetrahydro-naphtalen-2-yl)-allyloxy]-3-iodo-benzoate de méthyle

Huile jaune. Masse: 4,2 g. Rendement: 52%.

¹H NMR (CDCl₃): 1.22 (3H, s), 1.25 (3H, s), 1.62 à 1.66 (2H, m), 1.75 (3H, s), 1.77 à 1.83 (2H, m), 2.73 (2H, t, J=6.3Hz), 3.84 (3H, s), 4.54 (1H, d, J=8.8 Hz), 4.66 (1H, d, J=8.8 Hz), 6.89 (1H Ar, d, J=8.5 Hz), 6.9 à 7.00 (2H Ar, m), 7.25 (1H Ar, d, J=1.8 Hz), 7.72 (1H Ar, d, J=1.8 Hz), 7.93 (1H Ar, dd, J=8.4, J=1.9 Hz).

¹³C NMR (CDCl₃) d: 19.61 (CH₂), 26.41 (CH₃), 30.26 (CH₂ TTNN), 31.84 and 31.87 (CH₃ TTNN), 34.00 (C TTNN), 39.25 (CH₂ TTNN), 49.33 (C), 57.76 (OCH₃), 87.19 (CH₂O), 109.62 (CH Ar), 123.20 (C Ar), 123.51 (CH Ar), 124.32 (CH Ar), 126.13 (CH Ar), 129.20 (CH Ar), 131.23 (CH Ar), 134.70 (C Ar), 136.44 (C Ar), 142.92 (C Ar), 145.88 (C Ar), 163.68 (C-O Ar), 166.89 (COO).

### d) 3-(8,8-diméthyl-5,6,7,8-tetrahydro-naphtalen-2-yl)-3-méthyl-2,3-dihydro-benzofuran-5-carbaldehyde.

Le protocole expérimental est analogue à celui suivi pour l'exemple 8d appliqué au 3-(8,8-diméthyl-5,6,7,8-tetrahydro-naphtalen-2-yl)-3-méthyl-2,3-dihydro-benzofuran-5-carboxylate de méthyle.

Huile incolore. Masse: 1,7 g. Rendement: 93%.

¹H NMR (CDCl₃): 1.21 (3H, s), 1.26 (3H, s), 1.62 à 1.67 (2H, m), 1.76 (3H, s), 1.76 à 1.83 (2H, m), 2.73 (2H, t, J=6.2Hz), 4.58 (1H, d, J=8.9 Hz), 4.72 (1H, d, J=8.9 Hz), 6.91 à 7,01 (3H Ar,m), 7.24 (1H Ar, d, J=1.8 Hz), 7.57 (1H Ar, d, J=1.7 Hz), 7.74 (1H Ar, dd, J=1.8 Hz, J=8.3 Hz), 9.82 (1H, s).

### e) 3-[3-(8,8-diméthyl-5,6,7,8-tetrahydro-naphtalen-2-yl)-3-méthyl-2,3-dihydro-benzofuran-5-yl]-acrylate d'éthyle.

Le protocole expérimental est analogue à celui suivi pour l'exemple 1i appliqué au 3-(8,8-diméthyl-5,6,7,8-tetrahydro-naphtalen-2-yl)-3-méthyl-2,3-dihydro-benzofuran-5-carbaldehyde.

Huile incolore. Masse: 1,97 g. Rendement: 95%.
¹H NMR (CDCl₃): 1.22 à 1.33 (9H, m), 1.62 à 1.67 (2H, m), 1.74 (3H, s), 1.74 à 1.81 (2H, m), 2.73 (2H, t, J=6.4Hz), 4,21 (2H, q, J=7.1 Hz), 4.50 (1H, d, J=8.7 Hz), 4.62 (1H, d, J=8.7 Hz), 6.23 (1H, d, J=15.9 Hz), 6.88 (1H Ar, d, J=8.3 Hz), 6.96 à 7,01 (2H Ar,m), 7.20 (1H Ar, d, J=1.8 Hz), 7.26 (1H, s), 7.36 (1H Ar, dd,

J=1.8 Hz, J=8.3 Hz), 7.61 (1H Ar, d, J=15.9 Hz).

¹³C NMR (CDCl₃): 14.3, 19.6, 26.3, 30.3, 31.9, 34.0, 39.2, 49.5, 60.3, 87.0, 110.2, 115.2, 123.6, 124.4, 127.9, 129.2, 129.7, 134.8, 137.1, 142.9, 144.7, 145.9, 161.7, 167.4.

### EXEMPLE 13:

### Acide 3-[3-(8,8-diméthyl-5,6,7,8-tetrahydro-naphtalen-2-yl)-3-méthyl-2,3-dihydro-benzofuran-5-yl]-acrylique

Le protocole expérimental est analogue à celui suivi pour l'exemple 9 appliqué 3-[3-(8,8-diméthyl-5,6,7,8-tetrahydro-naphtalen-2-yl)-3-méthyl-2,3-dihydro-benzofuran-5-yl]-acrylate d'éthyle.

Solide blanc. Masse: 650 mg. Rendement: 97%. F=160°C.

¹H NMR (CDCl₃): 0.98 (3H, s), 1.01 (3H, s), 1.38 à 1.43 (2H, m), 1.51 (3H, s), 1.51 à 1.57 (2H, m), 2.49 (2H, t, J=6.1 Hz), 4.27 (1H, d, J=8.7 Hz), 4.38 (1H, d, J=8.7 Hz), 5.97 (1H, d, J=15.9 Hz), 6.63 (1H Ar, d, J=8.3 Hz), 6.68 à 6.77 (2H Ar, m), 6.97 (1H Ar, d, J=1.6 Hz), 7.00 (1H, s), 7.12 (1H, d, J=1.7 Hz, J=8.3 Hz), 7.33 (1H Ar, d, J=15.9 Hz).

¹³C NMR (CDCl₃): 20.1, 26.8, 30.1, 32.3, 34.4, 39.7, 49.9, 87.5, 110.8, 114.7, 124.0, 124.5, 124.8, 127.9, 129.7, 130.6, 135.2, 137.7, 143.2, 146.3, 147.5, 162.6, 173.5.

### EXEMPLE 14:

### 3-[3-(5,5-diméthyl-5,6,7,8-tetrahydro-naphtalen-2-yl)-3-méthyl-2,3-dihydro-benzofuran-5-yl]-acrylate d'éthyle.

### a) 5,6,7,8-tétrahydro-3-bromo-5,5-diméthyl-2-bromoacétonaphtone

Une solution de bromure de bromoacétyle (24,7 g, 0,28 mol) dans 200 ml de CH₂Cl₂ est additionnée goutte à goutte à une solution de chlorure d'aluminium (51,5 g, 0,39 mol) dans 100 ml de CH₂Cl₂ à 0°C. Le mélange est agité 1h à 0°C, puis une solution de 5,6,7,8-tétrahydro-3-bromo-5,5-diméthyl-2-acétonaphtone (60 g, 0,25 mol) dans le CH₂Cl₂ (100 ml) est additionnée goutte à goutte. L'agitation est poursuivie 2h puis le mélange est versé sur de l'eau et de la glace et extrait au CH₂Cl₂. La phase organique est lavée deux fois à l'eau, séchée sur sulfate de magnésium et concentrée à l'évaporateur rotatif sous vide.

Huile grise. Masse: 90 g. Rendement: quantitatif.

¹H NMR (CDCl₃): 1.28 (6H, s), 1.63 à 1.68 (2H, m), 1.76 à 1.84 (2H, m), 2.73 (2H, t, J=6 Hz), 3.88 (3H, s), 5.26 (2H, s), 6.72 (1H Ar, d, J=8.6 Hz), 7.24 (1H Ar, s), 7.53 (1H Ar, s), 7.97 (1H Ar, dd, J=2.0, Hz, J=8.6 Hz), 8.44 (1H Ar, d, J=2.0 Hz).

### b) 4-[2-(3-bromo-5,5-diméthyl-5,6,7,8-tetrahydro-naphtalen-2-yl)-2-oxo-ethoxy]-3-iodo-benzoate de méthyle

Le protocole expérimental est analogue à celui suivi pour l'exemple 1c appliqué à la 5,6,7,8-tétrahydro-3-bromo-5,5-diméthyl-2-bromoacétonaphtone et au 4-hydroxy-3-iodobenzoate de méthyle.

Solide jaune. Masse: 20 g. Rendement: 26%. F=142°C.

¹H NMR (CDCl₃): 1.28 (6H, s), 1.63 à 1.68 (2H, m), 1.76 à 1.84 (2H, m), 2.73 (2H, t, J=6 Hz), 3.88 (3H, s), 5.26 (2H, s), 6.72 (1H Ar, d, J=8.6 Hz), 7.24 (1H Ar, s), 7.53 (1H Ar, s), 7.97 (1H Ar, dd, J=2.0, Hz, J=8.6 Hz), 8.44 (1H Ar, d, J=2.0 Hz).

### c) 4-[2-(3-bromo-5,5-diméthyl-5,6,7,8-tetrahydro-naphtalen-2-yl)-allyloxy]-3-iodo-benzoate de méthyle.

Le protocole expérimental est analogue à celui suivi pour l'exemple 1d appliqué au 4-[2-(3-bromo-5,5-diméthyl-5,6,7,8-tetrahydro-naphtalen-2-yl)-2-oxo-ethoxy]-3-iodo-benzoate de méthyle.

Solide jaune. Masse: 5,6 g. Rendement: 32%. F=108°C.

¹H NMR (CDCl₃): 1.28 (6H, s), 1.62 à 1.66 (2H, m), 1.77 à 1.81 (2H, m), 2.69 (2H, t, J=6 Hz), 3.89 (3H, s), 4.86 (2H, s), 5.28 (1H, s), 5.70 (1H, s), 6.87 (1H Ar, d, J=8.7 Hz), 6.98 (1H Ar, s), 7.49 (1H Ar, s), 7.98 (1H Ar, dd, J=2.0 Hz, J=8.7 Hz), 8.45 (1H Ar, d, J=2.0 Hz).

### d) 3-(5,5-diméthyl-5,6,7,8-tetrahydro-naphtalen-2-yl)-3-méthyl-2,3-dihydro-benzofuran-5-carboxylate de méthyle.

Le protocole expérimental est analogue à celui suivi pour l'exemple 1e appliqué au 4-[2-(3-bromo-5,5-diméthyl-5,6,7,8-tetrahydro-naphtalen-2-yl)-allyloxy]-3-iodobenzoate de méthyle en doublant les équivalents molaire de tributylamine et d'acide formique.

Huile jaune. Masse: 4,2 g. Rendement: 52%.

¹H NMR (CDCl₃): 1.18 (6H, s), 1.55 à 1.59 (2H, m), 1.66 (3H, s), 1.69 à 1.74 (2H, m), 2.61 (2H, t, J=6 Hz), 3.78 (3H, s), 4.44 (1H, d, J=8.8 Hz), 4.61 (1H, d, J=8.8 Hz), 6.88 (1H Ar, d, J=8.4 Hz), 6.91 (1H Ar, s), 7.01 (1H Ar, dd, J=2.2 Hz, J=8.3 Hz), 7.23 à 7.27 (2H Ar, m), 7.74 (1H Ar, d, J=2.2 Hz), 7.93 (1H Ar, dd, J=8.4, J=2.2 Hz).

### e) 3-(5,5-diméthyl-5,6,7,8-tetrahydro-naphtalen-2-yl)-3-méthyl-2,3-dihydro- benzofuran-5-carbaldehyde.

Le protocole expérimental est analogue à celui suivi pour l'exemple 8d appliqué au 3-(5,5-diméthyl-5,6,7,8-tetrahydro-naphtalen-2-yl)-3-méthyl-2,3-dihydrobenzofuran-5-carboxylate de méthyle.

Huile incolore. Masse: 400 mg. Rendement: 62%.

¹H NMR (CDCl₃): ¹H NMR (CDCl₃): 1.18 (3H, s), 1.19 (3H, s), 1.53 à 1.58 (2H, m), 1.67 (3H, s), 1.67 à 1.74 (2H, m), 2.68 (2H, t, J=6.3 Hz), 4.48 (1H, d, J=9 Hz), 4.66 (1H, d, J=9 Hz), 6.76 à 6.96 (3H, m), 6.18 (1H Ar, d, J=8.3 Hz), 7.51 (1H Ar, d, J=1.8 Hz), 7.65 (1H Ar, dd, J=1.8 Hz, J=8.3 Hz), 9.75 (1H, s).

### f) 3-[3-(5,5-diméthyl-5,6,7,8-tetrahydro-naphtalen-2-yl)-3-méthyl-2,3-dihydro benzofuran-5-yl]-acrylate d'éthyle

Le protocole expérimental est analogue à celui suivi pour l'exemple 1i appliqué au 3-(5,5-diméthyl-5,6,7,8-tetrahydro-naphtalen-2-yl)-3-méthyl-2,3-dihydro- benzofuran-5-carbaldehyde.

Huile incolore. Masse: 400 mg. Rendement: 78%.

¹H NMR (CDCl₃): 1.26 à 1.33 (9H, s), 1.62 à 1.66 (2H, m), 1.72 (3H, s), 1.76 à 1.79 (2H, m), 2.72 (2H, t, J=6.2 Hz), 4.22 (2H, q, J=7 Hz), 4.48 (1H, d, J=8.8 Hz), 4.65 (1H, d, J=8.8 Hz), 6.24 (1H, d, J=15.9 Hz), 6.72 (1H Ar, d, J=8.3 Hz), 6.85 (1H Ar, s), 7.01 (1H Ar, d), 7.20 à 7.27 (2H Ar, m), 7.35 (1H, d, J=8.3 Hz), 7.61 (1H, d, J=15.9 Hz).

### EXEMPLE 15:

### Acide 3-[3-(5,5-diméthyl-5,6,7,8-tetrahydro-naphtalen-2-yl)-3-méthyl-2,3-dihydro - benzofuran-5-yl]-acrylique.

Le protocole expérimental est analogue à celui suivi pour l'exemple 9 appliqué au 3-[3-(5,5-diméthyl-5,6,7,8-tetrahydro-naphtalen-2-yl)-3-méthyl-2,3-dihydrobenzofuran-5-yl]-acrylate d'éthyle.

Solide blanc. Masse: 160 mg. Rendement: 43%. F=142°C.

¹H NMR (CDCl₃): 1.26 (6H, s), 1.62 à 1.66 (2H, m), 1.73 (3H, s), 1.72 à 1.79 (2H, m), 2.72 (2H, t, J=6.2 Hz), 4.49 (1H, d, J=8.8 Hz), 4.66 (1H, d, J=8.8 Hz), 6.25 (1H, d, J=15.9 Hz), 6.88 (1H Ar, d, J=8.3 Hz), 6.94 (1H Ar, s), 7.02 (1H Ar, d), 7.25 à 7.28 (2H Ar, m), 7.38 (1H, d, J=8.3 Hz), 7.71 (1H, d, J=15.9 Hz).

### EXEMPLE 16:

### 3-(3-méthyl-3-(5,5,8,8-tetraméthyl-5,6,7,8-tetrahydro-naphtalen-2-yl)-2,3-dihydro - benzofuran-6-yl]-acrylate d'éthyle.

### a) Acide 4-lodo-3-hydroybenzoïque.

On additionne goutte à goutte une solution de perchlorate de sodium à 3,6% à un mélange d' acide 4-hydroxybenzoïque (2,55 g, 18,5 mmol), de soude (0,74 g, 18,5 mmol), d' iodure de sodium (2,77 g, 18,5 mmol) dans le méthanol (50 ml), à 0°C. On laisse agiter deux heures à 0°C. On additionne 20 ml d'une solution de thiosulfate de sodium à 10%. Après agitation, on acidifie par de l'acide chlorydrique jusqu'à pH 1. On extrait par 100 ml d' ether éthylique. La phase organique est lavée deux fois par 80 ml d'eau, séchée sur sulfate de magnésium et concentrée à l'évaporateur rotatif sous vide à 40°C.

Solide blanc. Masse:2,71 g, Rendement: quantitatif. Pf: 178-185°C

RMN ¹H (DMSO, 250 MHz): 7,13 (1H Ar, dd, J=1,08 Hz, J=7,55Hz), 7,41 (1H Ar, d, J=1,08 Hz), 7,76 (1H Ar, d, J=7,55 Hz), 10,71 (1H, s), 12,96 (1H, s).

### b) 4-lodo-3-hydroybenzoate de méthyle.

On chauffe à reflux une solution d'acide 4-iodo 3-hydroxybenzoïque (2,71 g, 10 mmol) et d'acide sulfurique (0,7 ml) dans le méthanol (17 ml), pendant 6 h. On ajoute 20 ml d'eau et on alcalinise avec du bicarbonate de soude jusqu'à neutralité. On extrait par de l'éther éthylique (60 ml). La phase organique est lavée par deux fois 30 ml d'eau, séchée sur sulfate de magnésium, et concentrée à l'évaporateur rotatif sous vide à 40°C. Le produit est purifié par chromatographie flash sur colonne de silice (acétate d' éthyle 50 %, heptane 50 %).

Solide blanc. Masse: 2g. Rendement: 72%. Pf: 164°C

RMN ¹H (CDCl₃, 250 MHz): 3,91 (3H, s), 5,70 (1H,s), 7,33 (1H Ar, d, J=8,16Hz), 7,64 (1H Ar, s), 7,75 (1H Ar, d, J=8,16 Hz).

### c) 4-lodo-3-[2-oxo-2-(5,5,8,8-tetraméthyl-5,6,7,8-tetrahydro-naphtalen-2-yl)- ethoxy]-benzoate de méthyle.

Le protocole expérimental est analogue à celui suivi pour l'exemple 1c appliqué à la 5,6,7,8-tétrahydro-5,5,8,8-tétraméthy(-2-bromoacétonaphtone et au 3-hydroxy-4-iodobenzoate de méthylee.

Solide blanc. Masse: 1,69 g, Rendement: 81%. F: 124°C

RMN ¹H (CDCL₃, 250 MHz): 1,31 (6H, s),1,32 (6H, s), 1,71 (4H, s), 3,88 (3H, s), 5,42 (2H, s), 7,35 à 7,41 (2H Ar, m), 7,43 (1H Ar, d, J=8,25 Hz), 7,74 (1H Ar, dd, J=8,25,J=2,5 Hz), 7,90 (1H Ar, d, J=7,5 Hz)7,98 (1H Ar, d, J=2,5 Hz).

### d)4-lodo-3-[2-(5,5,8,8-tetraméthyl-5,6,7,8-tetrahydro-naphtalen-2-yl)-allyloxy]-benzoate de méthyle.

Le protocole expérimental est analogue à celui suivi pour l'exemple 1d appliqué au 4-lodo-3-[2-oxo-2-(5,5,8,8-tetraméthyl-5,6,7,8-tetrahydro-naphtalen-2-yl)-ethoxy]-benzoate de méthyle. Le produit est purifié par chromatographie flash sur colonne de silice (CH₂Cl₂ 60 %, heptane 40 %).

Solide blanc. Masse: 0,69 g, Rendement: 42%. Pf: 53°C

RMN ¹H (CDCL₃, 250 MHz): 1,29 (6H, s),1,30 (6H, s), 1,69 (4H, s), 3,91 (3H, s), 4,98 (2H, s), 5,58 (2H, s), 7,20 à 7,41 (4 H Ar, m), 7,50 (1H Ar, d, J=1,15 Hz), 7,87 (1H Ar, d, J=8,00).

RMN ¹³C (CDCL₃, 250 MHz): 31,34, 31,44, 33,71, 33,88, 34,55, 34,69, 51,86, 70,47, 92,84, 112,25, 113,66, 123,01, 123,06, 123,89, 131,08, 135,05, 139,12, 142,01, 144,53, 156,85, 166,05.

### e) 3-méthyl-3-(5,5,8,8-tetraméthyl-5,6,7,8-tetrahydro-naphtalen-2-yl)-2,3-dihydro-benzofuran-6-carboxylate de méthyle.

Le protocole expérimental est analogue à celui suivi pour l'exemple 1e appliqué au 4-lodo-3-[2-(5,5,8,8-tetraméthyl-5,6,7,8-tetrahydro-naphtalen-2-yl)-allyloxy]-benzoate de méthyle.

Le produit est purifié par chromatographie flash sur colonne de silice (CH₂Cl₂ 60% heptane 40 %)

Solide blanc. Masse: 330 mg, Rendement: 68%. Pf: 121°C

RMN ¹H (CDCL₃, 250 MHz): 1,20 (3H, s), 1,22 (3H, s), 1,25 (6H, s), 1,66 (4H, s), 1,73 (3H, s), 3,91 (3H, s), 4,48 (1H, d, J=8,75 Hz), 4,62 (1H, d, J=8,75 Hz), 7,00 (1H, dd, J=2 Hz, J=8,25 Hz), 7,09 (1H Ar, d, J=8 Hz), 7,18 à 7,24 (2 H Ar, m), 7,52 (1H Ar, s), 7,63 (1H Ar, d, J=8,00 Hz).

RMN ¹³C (CDCL₃, 250 MHz): 26,16, 31,77, 31,87, 33,96, 34,37, 35,01, 35,10, 49,79, 52,09, 86,48, 110,82, 122,84, 123,67, 123,93, 124,30, 126,60, 130,56, 141,35, 142,15, 143,32, 144,88, 159,80, 166,97.

### f) 3-méthyl-3-(5,5,8,8-tetraméthyl-5,6,7,8-tetrahydro-naphtalen-2-yl)-2,3-dihydro-benzofuran-6-carbaldehyde.

Le protocole expérimental est analogue à celui suivi pour l'exemple 8d appliqué au 3-méthyl-3-(5,5,8,8-tetraméthyl-5,6,7,8-tetrahydro-naphtalen-2-yl)-2,3-dihydrobenzofuran-6-carboxylate de méthyle.

Huile incolore. Masse: 1,25 g, Rendement: 90%.

RMN ¹H (CDCL₃, 250 MHz): 1,20 (3H, s), 1,23 (3H, s), 1,26 (6H, s), 1,66 (4H, s), 1,75 (3H, s), 4,51 (1H, d, J=8,7 Hz), 4,66 (1H, d, J=8,7 Hz), 7,00 (1H, dd, J=2,1 Hz, J=8,3 Hz), 7,16 à 7,25 (3H Ar, m), 7,36 (1 H Ar, d, J=1,3 Hz), 7,44 (1H Ar, dd, J=1,3 Hz, J=7,6 Hz).

### g) 3-[3-méthyl-3-(5,5,8,8-tetraméthyl-5,6,7,8-tetrahydro-naphtalen-2-yl)-2,3-dihydro-benzofuran-6-yl]-acrylate d'éthyle.

Le protocole expérimental est analogue à celui suivi pour l'exemple 1i appliqué au 3-méthyl-3-(5,5,8,8-tetraméthyl-5,6,7,8-tetrahydro-naphtalen-2-yl)-2,3-dihydrobenzofuran-6-carbaldehyde.

Huile incolore. Masse: 1,4 g. Rendement: 94%.

RMN ¹H (CDCL₃, 250 MHz): 1,21 (3H, s), 1,23 (3H, s), 1,26 (6H, s), 1,34 (3H, t, J=7,1 Hz), 1,66 (4H, s), 1,73 (3H, s), 4,26 (2H, t, J=7,1 Hz), 4,47 (1H, d, J=8,7 Hz), 4,61 (1H, d, J=8,7 Hz), 6,39 (1H, d, J=16 Hz), 6,98 à 7,06 (4H Ar, rn), 7,19 à 7,25 (2H Ar, m), 7,66 (1H, d, J=16 Hz) 9,95 (1H, s).

### EXEMPLE 17:

### Acide 3-[3-méthyl-3-(5,5,8,8-tetraméthyl-5,6,7,8-tetrahydro-naphtalen-2-yl)-2,3-dihydro-benzofuran-6-yl]-acrylique.

Le protocole expérimental est analogue à celui suivi pour l'exemple 9 appliqué 3-[3-méthyl-3-(5,5,8,8-tetraméthyl-5,6,7,8-tetrahydro-naphtalen-2-yl)-2,3-dihydro- benzofuran-6-yl]-acrylate d'éthyle.

Solide blanc. Masse: 1,05 g. Rendement: 80%. F=190°C.

RMN ¹H (CDCL₃, 250 MHz): 1,22 (3H, s), 1,23 (3H, s), 1,26 (6H, s), 1,66 (4H, s), 1,73 (3H, s), 4,48 (1H, d, J=8,7 Hz), 4,62 (1H, d, J=8,7 Hz), 6,42 (1H, d, J=16 Hz), 6,98 à 7,09 (4H Ar, m), 7,20 à 7,25 (2H Ar, m), 7,78 (1H, d, J=16 Hz).

### EXEMPLE 18:

### 3-[3-allyl-3-(5,5,8,8-tetraméthyl-5,6,7,8-tetrahydro-naphtalen-2-yl)-2,3-dihydro - benzofuran-5-yl]-acrylate d'éthyle

### a) 3-[3-allyl-3-(5,5,8,8-tetraméthyl-5,6,7,8-tetrahydro-naphtalen-2-yl)-2,3-dihydro-benzofuran-5-yl]-carboxylate de méthyle

On chauffe à 70°C pendant 8j un mélange, de diacétate de palladium (345 mg, 0,46 mmol), de tributylvinyl étain (1,3 ml, 4,56 mmol), de tributylamine (675 ul, 4,56 mmol) et de 3-lodo-4-[2-[5,6,7,8-tétrahydro-5,5,8,8-tétraméthyl-2-naphtyl]-1-propényloxy benzoate de méthyle (2,3 g, 4,56 mmol) dans l'acétonitrife (50 ml) en rajoutant du tributyl vinyl étain (0,8ml) toutes les 24 h. Le milieu réactionnel est concentré à l'évaporateur rotatif sous vide à 40°C, traité par de l'eau et de l'éther d'éthylique. Après décantation, la phase organique est lavée deux fois par 40 ml d'eau, séchée sur sulfate de magnésium, et concentrée à l'évaporateur rotatif sous vide à 40°C.

Le produit est purifié par chromatographie flash sur colonne de silice (CH₂Cl₂ 70% heptane 30 %) Huile incolore. Masse: 1,13g. Rendement 61%.

RMN ¹H (CDCL₃, 250 MHz): 1,22 à 1,26 (12H, m), 1,69 (4H, s), 2,88 (2H, t, J=7,3 Hz), 3,86 (3H, s), 4,63 (1H, d, J=9 Hz), 4,69 (1H, d, J=9 Hz), 5,03 (1H, s), 5,08 (1H, d, J=5,4), 5,58 (1H, m), 6,84 à 6,90 (2H Ar, m), 7,20 à 7,24 (2H Ar, m), 7,91 (1H Ar, d, J=1,9Hz), 7,93 (1H Ar, dd, J=1,8Hz, J=8,3Hz).

### b) 3-[3-allyl-3-(5,5,8,8-tetraméthyl-5,6,7,8-tetrahydro-naphtalen-2-yl)-2,3-dihydro-benzofuran-5-yl]-carbaldéhyde

Le protocole expérimental est analogue à celui suivi pour l'exemple 8d appliqué au 3-[3-Allyl-3-(5,5,8,8-tetraméthyl-5,6,7,8-tetrahydro-naphtalen-2-yl)-2,3-dihydro- benzofuran-5-yl]-carboxylate de méthyle. Le produit est purifié par chromatographie flash sur colonne de silice (AcOEt 10% heptane 90 %).

Huile incolore. Masse: 670 mg, Rendement: 39%.

RMN ¹H (CDCL₃, 250 MHz): 1,22 à 1,26 (12H, m), 1,67 (4H, s), 2,88 (2H, t, J=7,3 Hz), 4,68 (1H, d, J=9,1 Hz), 4,73 (1H, d, J=9,1 Hz), 5,04 (1H, s), 5,08 (1H, s), 5,57 (1H, m), 6,93 à 7,01 (2H Ar, m), 7,21 à 7,25 (2H Ar, m), 7,64 (1H Ar, d, J=1,7Hz), 7,25 (1H Ar, dd, J=1,8Hz, J=8,3Hz), 9,87 (1H, s).

### c) 3-[3-allyl-3-(5,5,8,8-tetraméthyl-5,6,7,8-tetrahydro-naphtalen-2-yl)-2,3-dihydro-benzofuran-5-yl]-acrylate d'éthyl

Le protocole expérimental est analogue à celui suivi pour l'exemple 1i appliqué au 3-[3-allyl-3-(5,5,8,8-tetraméthyl-5,6,7,8-tetrahydro-naphtalen-2-yl)-2,3-dihydrobenzofuran-5-yl]-carbaldéhyde. Le produit est purifié par chromatographie flash sur colonne de silice (AcOEt 80% heptane 80 %)

Huile incolore. Masse: 790 mg, Rendement: 99%.

RMN ¹H (CDCL₃, 250 MHz): 1,18 à 1,34 (15H, m), 1,67 (4H, s), 2,86 (2H, t, J=7,6 Hz), 4,23 (2H, q, J=7 Hz), 4,58 (1H, d, J=9 Hz), 4,64 (1H, d, J=9 Hz), 5,03 (1H, s), 5,08 (1H, d, J=3 Hz), 5,80 (1H, m), 6,25 (1H, d, 15,9Hz), 6,85 (1H Ar, d, J=8,3Hz), 7,00 (1H Ar, dd, J=2Hz, J=8,3Hz), 7,21 à 7,27 (3H Ar, m), 7,37 (1H Ar, dd, J=1,8Hz, J=8,3Hz), 7,67 (1H Ar, d, J=15,8Hz).

### EXEMPLE 19:

### Acide 3-[3-allyl-3-(5,5,8,8-tetraméthyl-5,6,7,8-tetrahydro-naphtalen-2-yl)-2,3-dihydro-benzofuran-5-yl]-acrylique

Une solution de 3-[3-allyl-3-(5,5,8,8-tetraméthyl-5,6,7,8-tetrahydro-naphtalen-2-yl)-2,3-dihydro- benzofuran-5-yl]-acrylate d'éthyle (790 mg, 1,78 mmol) et d'hydroxyde de sodium (720 mg, 17,8 mmol) dans le THF (20 ml) est chauffée 24h. à reflux. Le mélange est traité à l'eau et à l'acétate d'éthyle, acidifié par une solution d'acide chlorhydrique concentrée jusqu'à pH 1. Après décantation, la phase organique est lavée deux fois à l'eau, séchée sur sulfate de magnésium, et concentrée à l'évaporateur rotatif sous vide à 40°C. Le solide obtenu est lavé à l'heptane.

Solide blanc. Masse: 596 mg, Rendement: 80%. F=170°C.

RMN ¹H (CDCL₃, 250 MHz): 1,23 à 1,26 (12H, m), 1,67 (4H, s), 2,87 (2H, t, J=7,6 Hz), 4,60 (1H, d, J=9 Hz), 4,66 (1H, d, J=9 Hz), 5,04 (1H, s), 5,08 (1H, d, J=3 Hz), 5,58 (1H, m), 6,25 (1H, d, 15,8Hz), 6,87 (1H Ar, d, J=8,3Hz), 7,00 (1H Ar, d, J=8,3Hz), 7,22 à 7,29 (3H Ar, m), 7,40 (1H Ar, d, J=8,3Hz), 7,73 (1H, d, J=15,8Hz).

### EXEMPLE 20:

### (E)-3-[3-méthyl-3-(5,5,8,8-tetraméthyl-5,6,7,8-tetrahydro-naphtalen-2-yl)-2,3-dihydro-benzofuran-5-yl]-but-2-enaote de méthyle

### a) 1-[3-méthyl-3-(5,5,8,8-tetraméthyl-5,6,7,8-tetrahydro-naphtalen-2-yl)-2,3-dihydro-benzofuran-5-yl]-éthanone

Une solution de méthyllithium 1,6M dans l'éther éthylique (3,4 ml, 5,4 mmol) est additionnée à une solution d'acide 3-Méthyl-3-(5,5,8,8-tetraméthyl-5,6,7,8-tetrahydro-naphtalen-2-yl)-2,3-dihydro- benzofuran-5-carboxylique (exemple 2) (900 mg , 4,47 mmol) dans le THF (50ml) à -20°C. Le mélange est agité 4 h à -20°C puis est coulé sur de l'acétate d'éthyle et une solution d'acide chlorhydrique 1N. La phase organique est lavée 2 fois à l'eau, séchée sur sulfate de magnésium anhydre et concentrée à l'évaporateur rotatif sous vide à 40°C.

Huile incolore. Masse: 880 mg, Rendement: 98%.

RMN ¹H (CDCL₃, 250 MHz): 1,21 (3H, s), 1,24 (3H, s), 1,26 (6H, s), 1,66 (4H, s), 1,75 (3H, s), 2,53 (3H, s), 4,53 (1H, d, J=8,8 Hz), 4,68 (1H, d, J=8,7 Hz), 6,90 (1H Ar, d, J=8,3 Hz), 6,98 (1H Ar, dd, J=8,3 Hz,J=2 Hz), 7,20 à 7,25 (2H Ar, m), 7,69 (1H Ar, d, J=1,8 Hz), 7,86 (1H Ar, dd, J=8,3 Hz, J=2 Hz).

RMN ¹³C (CDCL₃, 250 Mhz): 26,5,, 31,8, 31,9, 34,0, 35,0, 35,1, 49,25, 87,3, 109,6, 123,8, 1124,2, 125,0, 126,7, 130,6, 131,2, 136,8, 142,2, 143,4, 144,9, 163,9, 196,7.

### b) (E)-3-[3-méthyl-3-(5,5,8,8-tetraméthyl-5,6,7,8-tetrahydro-naphtalen-2-yl)- 2,3-dihydro-benzofuran-5-yl]-but-2-enaote de méthyle.

Une solution de BuLi dans l'hexane 2,5 M (1,74, 4,3 mmol) est additionnée goutte à goutte à une solution de diisopropylamine (639 ul, 4,6 mmol), dans le THF (10ml) à -0°C. Le mélange est agité 15 mn à -0°C, puis le méthyl (triméthylsilyl)acétate (850 ul, 5,2 mmol) est additionné à -78°C. Le mélange est agité 15 mn à -78°C puis une solution de 1-[3-Méthyl-3-(5,5,8,8-tetraméthyl-5,6,7,8-tetrahydro-naphtalen-2-yl)-2,3-dihydro-benzofuran-5-yl]-ethanone (880 mg, 2,43 mmol) dans le THF (5 ml) est additionné à -78°C. Le milieu réactionnel est agité 1 h à -78°C puis traité par de l'acétate d'éthyle et une solution aqueuse de chlorure d'ammonium. Après décantation la phase organique est lavée deux fois à l'eau, séchée sur sulfate de magnésium anhydre et concentré à l'évaporateur rotatif sous vide à 40°C. Le produit est purifié par chromatographie flash sur colonne de silice (AcOEt 3%, heptane 97%).

Huile incolore. Masse: 460 mg, Rendement: 44%.

RMN ¹H (DMSO, 250 MHz): 1,17 (6H, s), 1,20 (6H, s), 1,60 (4H, s), 1,71 (3H, s), 2,49 (3H, s), 3,64 (3H, s), 4,47 (1H, d, J=8,9 Hz), 4,63 (1H, d, J=8,9 Hz), 6,12 (1H, s), 6,89 (1H Ar, d, J=9,2 Hz), 7,01 (1H Ar, dd), 7,20 à 7,26 (2H Ar, m), 7,43 à 7,46 (2H Ar, m).

### EXEMPLE 21:

### (Z)-3-[3-méthyl-3-(5,5,8,8-tetraméthyl-5,6,7,8-tetrahydro-naphtalen-2-yl)-2,3-dihydro-benzofuran-5-yl]-but-2-enoate de méthyle.

L'isomère (Z) est séparé de l'isomère (E) lors de la chromatographie flash sur colonne de silice (AcOEt 3%, heptane 97%).

Huile incolore. Masse: 130 mg, Rendement: 12%.

RMN ¹H (DMSO, 250 MHz): 1,16 (3H, s), 1,17 (3H, s), 1,20 (6H, s), 1,60 (4H, s), 1,67 (3H, s), 2,14 (3H, d, J=1,2 Hz), 3,41 (3H, s), 4,46 (1H, d, J=8,8 Hz), 4,59 (1H, d, J=8,8 Hz), 5,88 (1H, d, J=1,2 Hz), 6,82 (1H Ar, d, J=8,1 Hz), 7,00 à 7,10 (3H Ar, m), 7,18 (1H Ar, d, J=2,1 Hz), 7,24 (1H Ar, d, J=8,3 Hz).

### EXEMPLE 22:

### Acide (E)-3-[3-méthyl-3-(5,5,8,8-tetraméthyl-5,6,7,8-tetrahydro-naphtalen-2-yl)-2,3-dihydro-benzofuran-5-yl]-but-2-enoique

Le protocole expérimental est analogue à celui suivi pour l'exemple 9 appliqué au (E)-3-[3-méthyl-3-(5,5,8,8-tetraméthyl-5,6,7,8-tetrahydro-naphtalen-2-yl)-2,3-dihydro-benzofuran-5-yl]-but-2-enaote de méthyle.

Solide blanc. Masse: 370 mg. Rendement: 88%. F=110°C.

RMN ¹H (CDCL₃, 250 MHz): 1,21 (3H, s), 1,24 (3H, s), 1,26 (6H, s), 1,67 (4H, s), 1,74 (3H, s), 2,56 (3H, d, J=1Hz), 4,48 (1H, d, J=8,7 Hz), 4,60 (1H, d, J=8,7 Hz), 6,10 (1H, d, J=1Hz), 6,87 (1H Ar, d, J=8,4 Hz), 7,00 (1H Ar, dd, J=2,1 Hz, J=8,3 Hz), 7,20 à 7,22 (2H Ar, m), 7,39 (1H Ar, dd, J=2Hz, J=7,5 Hz).

RMN ¹³C (CDCL₃, 250 Mhz): 18,0, 26,1, 31,5, 31,6, 33,7, 34,1, 34,7, 34,8, 49,4, 86,6, 109,5, 113,8, 122,3, 123,5, 124,0, 126,4, 127,0, 134,5, 136,2, 142,1, 143,0, 144,6, 158,2, 160,9, 171,7.

### EXEMPLE 23

### 3-[3-méthyl-3-(5,5,8,8-tetraméthvl-5,6,7,8-tetrahydro-naphtalen-2-yl)-2,3-dihydro- benzo[b]thiophen-5-yl]-acrylate d'éthyle.

### a) 4-hydroxy-3-nitrobenzoate de méthyle.

Une solution d'acide 4-hydroxy-3-nitrobenzoïque (10 g, 54,6 mmol) et d'acide sulfurique concentré (1,8 ml) dans le méthanol (90 ml) est chauffée à reflux pendant 8 h. Le milieu réactionnel est traité par une solution de bicarbonate de soude. Après extraction à l'éther éthylique. La phase organique est lavée par deux fois à l'eau, séchée sur sulfate de magnésium, et concentrée à l'évaporateur rotatif sous vide à 40°C.

Solide jaune. Masse: 10,8 g, Rendement: 100%. F=74°C.

### b) 4-Diméthylthiocarbamoyloxy-3-nitrobenzoate de méthyle.

Du NaH à 80% (1,98g, 65,52 mmol) est additionné à une solution 4-hydroxy-3-nitrobenzoate de méthyle (10,5g, 53,25 mmol) dans le DMF (50ml) à 0°C. L'agitation est poursuivie pendant 30 mn puis une solution de chlorure de diméthylthiocarbamoyle (8,56g, 69,2 mmol) dans le DMF (50 ml) est additionnée goutte à goutte à 0°C. Le mélange est agité 24h à température ambiante, traité par une solution aqueuse de NH4Cl et de l'éther éthylique. La phase organique est lavée par deux fois à l'eau, séchée sur sulfate de magnésium, et concentrée à l'évaporateur rotatif sous vide à 40°C. Le produit est purifié par chromatographie flash sur colonne de silice (acétate d'éthyle 30 %, heptane 70 %).

Solide blanc. Masse: 10,6 g, Rendement: 70%. F=99°C.

### c) 4-Diméthylcarbamoylsulfanyl-3-nitrobenzoate de méthyle.

Le 4-Diméthylthiocarbamoyloxy-3-nitrobenzoate de méthyle (10,6g, 37,3 mmol) est chauffé à 180°C pendant 15 mn.

Solide jaune. Masse: 10,6 g, Rendement: 100%. F=79°C.

### d) 3-amino-4-diméthylcarbamoylsulfanyl-benzoate de méthyle.

Une solution d'acide chlorhydrique concentrée est additionnée goutte à goutte à 0°C sur un mélange de fer (12,7 g) et de 4-Diméthylthiocarbamoyloxy-3-nitrobenzoate de méthyle (10,6g, 37,3 mmot) dans l'éthanol à 0°C. Le milieu réactionnel est agité à température ambiante 5h, puis traité par une solution de bicarbonate de soude. Après extraction au dichlorométhane, la phase organique est lavée par deux fois à l'eau, séchée sur sulfate de magnésium, et concentrée à l'évaporateur rotatif sous vide à 40°C. Le produit est purifié par chromatographie flash sur colonne de silice (acétate d'éthyle 50 %, heptane 50 %).

Solide jaune. Masse: 7,1 g, Rendement: 75%. F=135°C.

### e) 4-diméthylcarbamoylsulfanyl-3-iodo-benzoate de méthyle.

Une solution de 3-Amino-4-diméthylcarbamoylsulfanylbenzoate de méthyle (5,98 g, 23,5 mmol) d'isopentyl nitrite ( 16,2 ml) dans le diiodométhane (138 ml) est chauffé à 70°C pendant 2h. Le diiodométhane est distillé à 10-1 Atm.; puis le produit est purifié par chromatographie flash sur colonne de silice (CH₂Cl₂ 90 %, heptane 10%).

Solide jaune. Masse: 3,45 g, Rendement: 43%. F=72°C.

### f) 3-lodo-4-mercaptobenzoate de méthyle.

Un mélange de 4-diméthylcarbamoylsulfanyl-3-iodobenzoate de méthyle (3,9g, 16,6 mmol), de carbonate de potassium (2g,) dans le méthanol est agité 12 h à température ambiante. Le mélange est traité par une solution d'acide chlorhydrique concentré qsp pH=1. Après extraction au dichlorométhane, la phase organique est lavée par deux fois à l'eau, sèchée sur sulfate de magnésium, et concentrée à l'évaporateur rotatif sous vide à 40°C.

Solide jaune. Masse: 2,7 g, Rendement: 100%. F=52°C.

### g) 2-(5,5,8,8-tetraméthyl-5,6,7,8-tetrahydro-naphtalen-2-yl)-acrylate de méthyle.

Du NaH à 75% (45 mg, 1,4 mmol) est additionné à une solution d'acide 2-(5,5,8,8-Tetraméthyl-5,6,7,8-tetrahydro-naphtalen-2-yl)-acrylique (300 mg, 1,16 mmol) {Synthèse décrite dans le brevet WO 9206948} dans le DMF (10ml).

L'agitation est poursuivie pendant 1 h. à température ambiante, puis du iodomethane (87 ul, 1,4 mmol) est additionnée goutte à goutte. Le mélange est agité 1h. à température ambiante, traité par de l'eau et de l'éther éthylique. La phase organique est lavée par deux fois à l'eau, séchée sur sulfate de magnésium, et concentrée à l'évaporateur rotatif sous vide à 40°C. Le produit est purifié par chromatographie flash sur colonne de silice (acétate d'éthyle 20 %, heptane 80 %).

Solide jaune. Masse: 45 mg, Rendement: 95%.

RMN ¹H (CDCL₃, 250 MHz): 1,28 (6H, s),1,29 (6H, s), 1,69 (4H, s), 3,82 (3H, s), 5,87 (1H, d, J=1,3Hz), 6,30 (1H, d, J=1,3 Hz), 7,21 (1H Ar, dd, J=2 Hz, J=8 Hz), 7,29 (1H Ar, d, J=8 Hz), 7,35 (1H Ar, d, J=2 Hz).

### h) 2-(5,5,8,8-tetraméthyl-5,6,7,8-tetrahydro-naphtalen-2-yl)-prop-2-en-1-ol

Une solution 1M d'hydrure de diisobutylaluminium dans le toluène (2,78 ml, 2,78 mmol) est additionnée à-78°C, goutte à goutte à une solution de 2-(5,5,8,8-Tetraméthyl-5,6,7,8-tetrahydro-naphtalen-2-yl)-acrylate de méthyle (310 mg, 1,14 mmol) dans le dichloromethane (3 ml). La solution est agitée 1h à 0°C, puis traité par une solution de tartrate double de sodium et filtré sur silice.

Huile incolore. Rendement: 65%.

RMN ¹H (CDCL₃, 250 MHz): 1,28 (6H, s),1,30 (6H, s), 1,77 (4H, s), 4,53 (2H, d, J=5,4Hz), 5,29 (1H, s), 5,43 (1H, s), 7,21 (1H Ar, dd, J=1,9Hz, J=8,2Hz), 7,29 (1H Ar, d, J=8,2Hz), 7,38 (1H Ar, d, J=1,9Hz).

### i) 6-(1-bromométhyl-vinyl)-1,1,4,4-tetraméthyl-1,2,3,4-tetrahydro-naphtalene

Du tétrabromométhane (1,21 g, 3,8 mmol) est additionné à un mélange de triphénylphosphine (970 mg, 3,8 mmol) dans l'éther éthylique (20 ml). Une solution de 2-(5,5,8,8-Tetraméthyl-5,6,7,8-tetrahydro-naphtalen-2-yl)-prop-2-en-1-ol (300 mg, 1,2 mmol) dans l'éther éthylique (2 ml) est additionnée. L'agitation est poursuivie 24h, puis le milieu réactionnel est traité par de l'eau et de l'acétate d'éthyle. La phase organique est lavée à l'eau puis concentrée à l'évaporateur rotatif sous vide. L'huile obtenue est reprise dans l'heptane et filtrée sur silice. Le filtrat est concentré à l'évaporateur rotatif sous vide.

Huile jaune. Rendement: quantitatif.

RMN ¹H (CDCL₃, 250 MHz): 1,28 (6H, s),1,31 (6H, s), 1,78 (4H, s), 4,37 (2H, s), 5,44 (1H, s), 5,55 (1H, s), 7,23 à 7,28 (2H Ar, m), 7,44 (1H Ar, s).

### j) 3-lodo-4-[2-(5,5,8,8-tetraméthyl-5,6,7,8-tetrahydro-naphtalen-2-yl)-allylsulfanyl]-benzoate de méthyle.

Du NaH à 75% (115 mg, 3,58 mmol) est additionné à une solution de 3-lodo-4-mercapto-benzoate de méthyle (531 mg, 3,25 mmol) dans le DMF (20ml) à 0°C. L'agitation est poursuivie pendant 20 mn à température ambiante, puis une solution de 6-(1-bromométhyl-vinyl)-1,1,4,4-tetraméthyl-1,2,3,4-tetrahydro- naphtalene (1 g, 3,25 mmol) dans le DMF (5 ml) est additionnée goutte à goutte. Le mélange est agité 2h à température ambiante, traité par une solution aqueuse d'acide chlorhydrique et d'éther éthylique. La phase organique est lavée deux fois à l'eau, séchée sur sulfate de magnésium, et concentrée à l'évaporateur rotatif sous vide à 40°C. Le produit est purifié par chromatographie flash sur colonne de silice (CH₂Cl₂ 50% heptane 50 %).

Solide blanc. Masse: 680 mg, Rendement: 54%. F=142°C.

RMN ¹H (CDCL₃, 250 MHz): 1,28 (6H, s),1,30 (6H, s), 1,69 (4H, s), 3,89 (3H, s), 4,02 (2H, s), 5,37 (1H, s), 5,51 (1H, s), 7,18 (1H Ar, d, J=8,3Hz), 7,21 à 7,31 (2H Ar, m), 7,39 (1H Ar, d, J=1,7 Hz), 7,91 (1H Ar, dd, J=8,3 Hz,J=1,7 Hz), 8,43 (1H Ar, d, J=1,7 Hz).

RMN ¹³C (CDCL₃, 250 MHz): 32,1, 32,2, 34,5, 34,7, 35,3, 35,4, 38,9, 52,6, 97,5, 115,9, 123,6, 124,5, 126,2, 127,0, 128,2, 129,7, 136,8, 140,5, 141,8, 145,3, 145,4, 148,7, 165,8.

### k) 3-méthyl-3-(5,5,8,8-tetraméthyl-5,6,7,8-tetrahydro-naphtalen-2-yl)-2,3-dihydro-benzo[b]thiophene-5-carboxylate de méthyle.

On chauffe à 80°C pendant 4 h un mélange de tributhylamine (881 ul, 3,7 mmol), de tétrakis(triphénylphosphine)palladium (367 mg, 0,32 mmol), d'acide formique (63 ul, 1,7 mmol) et de 3-lodo-4-[2-(5,5,8,8-tetraméthyl-5,6,7,8-tetrahydro- naphtalen-2-yl)-allylsulfanyl]-benzoate de méthyle (655 mg, 1,7 mmol) dans l'acétonitrile (25 ml). Le milieu réactionnel est concentré à l'évaporateur rotatif sous vide à 40°C traité par de l'eau et de l'éther d'éthylique. Après décantation, la phase organique est lavée deux fois à l'eau, séchée sur sulfate de magnésium, et concentrée à l'évaporateur rotatif sous vide à;40°C.

Le produit est purifié par chromatographie flash sur colonne de silice (CH₂Cl₂ 50% heptane 50 %)

Huile incolore. Masse: 121 mg, Rendement: 19%.

RMN ¹H (CDCL₃, 250 MHz): 1,19 (3H, s), 1,23 (3H, s), 1,26 (6H, s), 1,66 (4H, s), 1,76 (3H, s), 3,39 (1H, d, J=11 Hz), 3,64 (1H, d, J=11 Hz), 3,84 (3H, s), 6,98 (1H Ar, dd, J=2 Hz, J=8,4 Hz), 7,19 à 7,24 (2H Arm), 7,29 (1H Ar, d, J=8,1 Hz), 7,55 (1H Ar, d, J=1,6 Hz), 7,85 (1H Ar, dd, J=1,7,J=8,1 Hz).

RMN ¹³C (CDCL₃, 250 MHz): 26,38, 32,3, 32,4, 34,40, 34,8, 35,5, 35,6, 49,7, 52,3, 55,2, 122,4, 124,4, 125,1, 126,6, 126,9, 127,0, 129,7, 143,1, 143,8, 145,1, 148,4, 148,6, 167,4.

### I) 3-méthyl-3-(5,5,8,8-tetraméthyl-5,6,7,8-tetrahydro-naphtalen-2-yl)-2,3-dihydro-benzo[b]thiophene-5-méthanol.

Une solution 1M d'hydrure de diisobutylaluminium dans le toluène (0,67 ml, 0,67 mmol) est additionnée à 0°C, goutte à goutte à une solution de 3-méthyl-3-(5,5,8,8-tetraméthyl-5,6,7,8-tetrahydro-naphtalen-2-yl)-2,3-dihydro- benzo[b]thiophene-5-carboxylate de méthyle (121 mg, 0,31 mmol) dans le toluène (5 ml). La solution est agitée 2h à 0°C, puis traité par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.

Huile incolore. Masse: 120 mg, Rendement: quantitatif.

### m) 3-méthyl-3-(5,5,8,8-tetraméthyl-5,6,7,8-tetrahydro-naphtalen-2-yl)-2,3-dihydro-benzo[b]thiophene-5-carbaldehyde.

Un mélange d'alcool précedement obtenu (120 mg, 0,31 mmol), d'oxyde de manganèse (IV) (270 mg, 3,1 mmol) dans le dichlorométhane (5 ml) est agité à température ambiante 3h. L'oxyde de manganèse est éliminé par filtration sur silice. Le produit est obtenu par concentration à l'évaporateur rotatif sous vide à 40°C.

Solide blanc. Masse:100 mg, Rendement: 88%. F=143°C.

RMN ¹H (CDCL₃, 250 MHz): 1,11 (3H, s), 1,17 (3H, s), 1,20 (6H, s), 1,60 (4H, s), 1,79 (3H, s), 3,32 (1H, d, J=11,3 Hz), 3,63 (1H, d, J=11,3 Hz), 6,93 (1H Ar, dd, J=2,2 Hz, J=8,2 Hz), 7,14 à 7,18 (2H Ar, m), 7,28 (1H, d, J=1,5Hz), 7,31 (1H Ar, d, J=8 Hz), 7,59 (1H Ar, dd, J=1,5, J=8 Hz), 9,76 (1H, s).

### n) 3-[3-méthyl-3-(5,5,8,8-tetraméthyl-5,6,7,8-tetrahydro-naphtalen-2-yl)-2,3-dihydro-benzo[b]thiophen-5-yl]-acrylate d'éthyle.

Le protocole expérimental est analogue à celui suivi pour l'exemple 1i appliqué au 3-Méthyl-3-(5,5,8,8-tetraméthyl-5,6,7,8-tetrahydro-naphtalen-2-yl)-2,3-dihydrobenzo[*b*]thiophene-5-carbaldehyde. Le produit est purifié par filtration sur silice (CH₂Cl₂).

Huile incolore. Masse: 115 mg, Rendement: quantitatif.

RMN ¹H (CDCL₃, 250 MHz): 1,19 à 1,33 (15H, m), 1,67 (4H, s), 1,74 (3H, s), 3,35 (1H, d, J=11 Hz), 3,65 (1H, d, J=11 Hz), 4,22 (2H, q, J=7,1Hz), 6,27 (1H, d, J=15,9 Hz), 6,99 à 7,03 (2H Ar, m), 7,21 à 7,27 (3H Ar, m), 7,33 (1H Ar, d, J=8,1Hz), 7,57 (1H, dd, J=1,5, J=8 Hz).

### EXEMPLE 24

### Acide 3-[3-méthyl-3-(5,5,8,8-tetraméthyl-5,6,7,8-tetrahydro-naphtalen-2-yl)-2,3-dihydro-benzo[b]thiophen-5-yl]-acrylique.

Le protocole expérimental est analogue à celui suivi pour l'exemple 9 appliqué au 3-[3-méthyl-3-(5,5,8,8-tetraméthyl-5,6,7,8-tetrahydro-naphtalen-2-yl)-2,3-dihydro-benzo[*b*]thiophen-5-yl]-acrylate d'éthyle.

Solide blanc. Masse: 70 mg, Rendement: 75%. F=196°C.

RMN ¹H (CDCL₃, 250 MHz): 1,19 (3H, s), 1,25 (3H, s), 1,27 (6H, s), 1,67 (4H, s), 1,75 (3H, s), 3,35 (1H, d, J=11,2 Hz), 3,66 (1H, d, J=11,2 Hz), 6,26 (1H, d, J=15,9 Hz), 7,00 à 7,03 (2H Ar, m), 7,21 à 7,37 (4H Ar, m), 7,67 (1H Ar, d, J=15,9Hz).

### EXEMPLE 25

### 3-[3-(5,6,7,8-tetrahydro-5,5,8,8-tetraméthyl-2-naphtyl)-7-méthoxy-3-méthyl-2,3-dihydro-benzofuran-5-yl]-acrylate d'éthyle

### a) 3-lodo-5-methoxy-4-[2-(5,5,8,8-tetraméthyl-5,6,7,8-tetrahydro-naphtalen-2-yl)-allyloxy]-benzaldehyde.

Le protocole expérimental est analogue à celui suivi pour l'exemple 23 j appliqué à la 5-iodovaniline et au 6-(1-bromométhyl-vinyl)-1,1,4,4-tetraméthyl-1,2,3,4-tetrahydro-naphtalene. Le produit est purifié par chromatographie flash sur colonne de silice (CH₂Cl₂ 50% heptane 50 %).

Solide jaune. Masse: 1,05 g, Rendement: 65%. F=75°C

RMN ¹H (CDCL₃, 250 MHz): 1,28 (6H, s),1,29 (6H, s), 1,69 (4H, s), 3,91 (3H, s), 4,99 (2H, s), 5,55 (1H, s), 5,59 (1H, s), 7,29 (2H Ar, s), 7,41 à 7,45 (2H Ar, m), 7,84 (1H Ar, d, J=1,8 Hz), 9,83 (1H s).

### b) 3-(5,6,7,8-tetrahydro-5,5,8,8-tetraméthyl-2-naphtyl)-7-methoxy-3-méthyl-2,3-dihydro-benzofuran-5-carbaldehyde.

Le protocole expérimental est analogue à celui suivi pour l'exemple 1e appliqué au 3-lodo-5-methoxy-4-[2-(5,5,8,8-tetraméthyl-5,6,7,8-tetrahydro-naphtalen-2-yl)-allyloxy]-benzaldehyde. Le produit est purifié par chromatographie flash sur colonne de silice (CH₂Cl₂ 50% heptane 50 %).

Huile jaune. Masse: 500 mg, Rendement: 67%.

¹H (CDCl₃): 1,20 à 1,26 (12H, m), 1,67 (4H, s), 1,77 (3H, s), 3,98 (3H, s), 4,64 (1H, d, J=8,9Hz), 4,77 (1H, d, J=8,9Hz), 6,99 (1H, dd, J=2,2Hz, J=8,2Hz), 7,20 à 7,26 (3H Ar, m), 7,37 (1H Ar, d, J=1,3Hz), 9,79 (1H, s).

### c) 3-[3-(5,6,7,8-tetrahydro-5,5,8,8-tetraméthyl-2-naphtyl)-7-méthoxy-3-méthyl-2,3-dihydro-benzofuran-5-yl]-acrylate d'éthyle

Le protocole expérimental est analogue à celui suivi pour l'exemple 1i appliqué au 3-(5,6,7,8-tetrahydro-5,5,8,8-tetraméthyl-2-naphtyl)-7-méthoxy-3-méthyl-2,3-dihydro-benzofuran-5-carbaldéhyde. Le produit est purifié par filtration sur silice (CH₂Cl₂).

Huile incolore. Masse: 420 mg, Rendement: 71%.

¹H (CDCl₃): 1,21 à 1,33 (15H, m), 1,67 (4H, s), 1,73 (3H, s), 3,94 (3H, s), 4,23 (2H, q, J=7,1Hz), 4,55 (1H, d, J=8,8Hz), 4,69 (1H, d, J=8,8Hz), 6,26 (1H, d, 15,9Hz), 6,85 (1H Ar, s), 6,96 à 7,02 (2H Ar, m), 7,20 à 7,26 (2H Ar, m), 7,60 (1H Ar, d, J=15,9Hz).

### EXEMPLE 26:

### Acide 3-[3-(5,6,7,8-tetrahydro-5,5,8,8-tetraméthyl-2-naphtyl)-7-méthoxy-3-méthyl-2,3-dihydro-benzofuran-5-yl]-acrylique.

Le protocole expérimental est analogue à celui suivi pour l'exemple 9 appliqué au 3-[3-(5,6,7,8-tetrahydro-5,5,8,8-tetraméthyl-2-naphtyl)-7-méthoxy-3-méthyl-2,3-dihydro-benzofuran-5-yl]-acrylate d'éthyle. Le produit est purifié par cristallisation.

Solide blanc. Masse: 350 mg, Rendement: 89%.F=65°C.

¹H (CDCl₃): 1,21 à 1,26 (12H, m), 1,67 (4H, s), 1,74 (3H, s), 3,95 (3H, s), 4,57 (1H, d, J=8,8Hz), 4,70 (1H, d, J=8,8Hz), 6,25 (1H, d, 15,9Hz), 6,88 (1H Ar, s), 6,99 à 7,03 (2H Ar, m), 7,21 à 7,26 (2H Ar, m), 7,69 (1H Ar, d, J=15,9Hz).

### EXEMPLE 27:

### N-(4-hydroxy-phenyl)-3-[7-methoxy-3-méthyl-3-(5,5,8,8-tetraméthyl-5,6,7,8-tetrahydro-naphtalen-2-yl)-2,3-dihydro-benzofuran-5-yl]-acrylamide

Une solution de Acide 3-[3-(5,6,7,8-tetrahydro-5,5,8,8-tetraméthyl-2-naphtyl)-7-méthoxy-3-méthyl-2,3-dihydro-benzofuran-5-yl]-acrylique (150 mg, 0.357 mmol), de 1-hydroxybenzotriazole (96 mg, 0,714 mmol), de 1,3-dicyclohexylcarbodiimide (147 mg, 0.714 mmol) et de 4-aminophenol (39 mg, 0.357 mmol) dans 5 ml de THF et 5 ml de DMF, est agitée à température ambiante pendant 15 heures. On ajoute de l'eau et de l'acetate d'éthyle. Après agitation et décantation, la phase aqueuse est extraite avec de l'acetate d'éthyle. Les phases organiques sont alors réunies et lavées deux fois à l'eau, puis séchées sur sulfate de magnésium et concentrées à l'évaporateur rotatif sous vide à 40°C. Le produit est alors purifié par chromatographie flash sur colone de silice ( heptane 50 %, acetate d'éthyle 50 %)

Solide blanc. Masse 70 mg. Rendement 37%. F=80°C.

¹H (CDCl₃): 1,21 à 1,25 (12H, m), 1,66 (4H, s), 1,73 (3H, s), 3,92 (3H, s), 4,54 (1H, d, J=8,8Hz), 4,67 (1H, d, J=8,8Hz), 6,34 (1H, d, 15,4Hz), 6,77 (2H Ar, d, J=8,7Hz), 6,87 (1H Ar, s), 6,94 (1H Ar, s), 7,01 (1H Ar, d, J=8,2 Hz), 7,20 à 7,24 (2H Ar, m), 7,32 à 7,36 (2H Ar, m), 7,63 (1H Ar, d, J=15,4Hz).

### B. EXEMPLES DE FORMULATION

### 1) VOIE ORALE

(a) On prépare la composition suivante sous la forme d'un comprimé de 0,8 g

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administrera à un individu adulte 1 à 3 comprimés par jour pendant 3 à 6 mois selon la gravité du cas traité.

(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle Arôme q.s. | 0,040 g |
| Eau purifiée q.s.p | 5 ml |

Pour le traitement de l'acné, on administrera à un individu adulte 1 ampoule par jour pendant 3 mois selon la gravité du cas traité.

(c) On prépare la formulation suivante destinée à être conditionnée en gélules :

| | |
|---|---|
| Composé de l'exemple 1 | 0,025 g |
| Amidon de maïs | 0,060 g |
| Lactose q.s.p | 0,300 g |

Les gélules utilisées sont constituées de gélatine, d'oxyde de titane et d'un conservateur.

Dans le traitement du psoriasis, on administrera à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 2 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 jours.

(b) On prépare un gel en réalisant la formulation suivante :

| | |
|---|---|
| Composé de l'exemple 1 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p | 100,000 g |

Ce gel sera appliqué sur une peau atteinte de dermatose ou une peau acnéique 1 à 3 fois par jour pendant 6 à 12 semaines selon la gravité du cas traité.

(c) On prépare une lotion antiséborrhéique en procédant au mélange des ingrédients suivants :

| | |
|---|---|
| Composé de l'exemple 2 | 0,030 g |
| Propylène glyco | 5,000 g |
| Butylhydroxytoluène | 0,100 g |
| Ethanol (à 95°) q.s.p | 100,000 g |

Cette lotion sera appliquée deux fois par jour sur un cuir chevelu séborrhéique et on constate une amélioration significative dans un délai compris entre 2 et 6 semaines.

(d) On prépare une composition cosmétique contre les effets néfastes du soleil en procédant au mélange des ingrédients suivants :

| | |
|---|---|
| Composé de l'exemple 1 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition sera appliquée quotidiennement, elle permet de lutter contre le vieillissement photoinduit.

(e) On prépare la crème Huile dans l'Eau non ionique suivante :

| | |
|---|---|
| Composé de l'exemple 2 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 Jours.

(f) On prépare un gel topique en procédant au mélange des ingrédients suivants :

| | |
|---|---|
| Composé de l'exemple 1 | 0,050 g |
| Ethanol | 43,000 g |
| α -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel sera appliqué dans le traitement de l'acné 1 à 3 fois par jour pendant 6 à 12 semaines selon la gravité du cas traité.

(g) On prépare une lotion capillaire anti-chute et pour la repousse des cheveux en procédant au mélange des ingrédients suivants :

| | |
|---|---|
| Composé de l'exemple 2 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On appliquera cette lotion 2 fois par jour pendant 3 mois sur un cuir chevelu ayant subi une chute de cheveu importante.

(h) On prépare une crème anti-acnéique en procédant au mélange des ingrédients suivants :

| | |
|---|---|
| Composé de l'exemple 1 | 0,050 g |
| Acide rétinoïque | 0,010 g |
| Mélange de stéarates de glycérol et de polyéthylène glycol (75 moles) vendu sous le nom de "Gelot 64" ..par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000g |

Cette crème sera appliquée sur une peau atteinte de dermatose ou une peau acnéique 1 à 3 fois par jour pendant 6 à 12 semaines.

(i) On prépare une crème huile dans l'eau en réalisant la formulation suivante :

| | |
|---|---|
| Composé de l'exemple 1 | 0,020 g |
| 17-valérate de bétamethasone | 0,050 g |
| S-carboxyméthyl cystéine | 3,000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000g |

Cette crème sera appliquée 2 fois par jour sur une peau atteinte de dermatose pendant 30 jours.

(j) On prépare la crème de type huile dans l'eau suivante :

| | |
|---|---|
| Acide lactique | 5,000 g |
| Composé de l'exemple 1 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylèrie glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812", par la société "DYNAMIT NOBEL" | 4,000 g |
| Eau q.s.p | 100,000g |

Cette crème sera appliquée 1 fois par jour, elle aide à lutter contre le vieillissement qu'il soit photoinduit ou chronologique.

## Revendications

1. Composés, caractérisés par le fait qu'ils répondent à la formule générale (I) suivante : 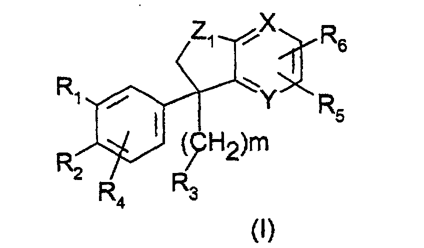 dans laquelle :
Z₁ représente un atome ou un radical choisi parmi : O, S et NR',
X et Y, identiques ou différents, représentent CH ou N, étant entendu que X et Y ne peuvent être simultanément des atomes d'azote,
R₁ et R₂ pris ensemble forment avec le cycle aromatique adjacent un cycle à 5 ou 6 chainons éventuellement substitué par des groupes méthyle et /ou éventuellement interrompu par un un radical SO, un radical SO₂, un atome d'oxygène ou de soufre,
R₃ représente :
(i) un atome d'hydrogène, un radical alkyle ayant de 1 à 6 atomes de carbone linéaire ou ramifié éventuellement substitué par un ou plusieurs atomes d'halogène, un radical alkényle ayant de 2 à 6 atomes de carbone linéaire ou ramifié, un radical alkynyle ayant de 3 à 6 atomes de carbone linéaire ou ramifié, un radical aryle, un radical monohydroxyalkyle ou polyhydroxyalkyle, un radical polyéther, un radical cyano ou un radical -O-R₇,
R₇ ayant la signification donnée ci-après,
(ii) un radical de formule : 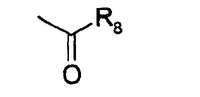 R₈ ayant la signification donnée ci-après,
ou (iii) un radical de formule : 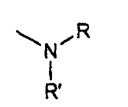 R et R' ayant la signification donnée ci-après
R₄ représente :
(i) un atome d'hydrogène,
(ii) un radical alkyle ayant de 1 à 6 atomes de carbone linéaire ou ramifié éventuellement substitué par un ou plusieurs atomes d'halogène,
(iii) un atome d'halogène,
(iv) un radical -OR₇,
R₇ ayant la signification donnée ci-après,
R₅ représente :
(i) un radical de formule : 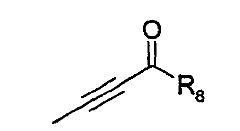
(ii) un radical de formule : 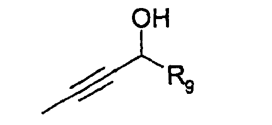
(iii) un radical de formule : 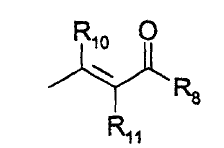
(iv) un radical de formule : 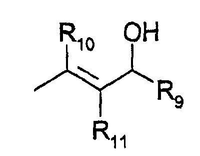
(v) un radical de formule : 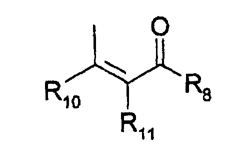
(vi) un radical de formule : 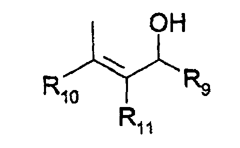 R₈, R₉, R₁₀ et R₁₁ ayant les significations données ci-après,
R₆ représente un atome d' hydrogène, un atome d' halogène, un radical alkyle ayant de 1 à 6 atomes de carbone linéaire ou ramifié éventuellement substitué par un ou plusieurs atomes d'halogène ou le radical -OR₇,
R₇ ayant la signification donnée ci-après,
R₇, identique ou différent, représente un atome d'hydrogène, un radical alkyle ayant de 1 à 6 atomes de carbone linéaire ou ramifié éventuellement substitué par un ou plusieurs atomes d'halogène, un radical aryle, un radical aralkyle, éventuellement substitué(s), un radical monohydroxyalkyle ou polyhydroxyalkyle, un radical polyéther ou un radical acyle ayant de 1 à 6 atomes de carbone linéaire ou ramifié,
R₈ représente :
(a) un atome d' hydrogène, un radical alkyle ayant de 1 à 6 atomes de carbone linéaire ou ramifié éventuellement substitué par un ou plusieurs atomes d'halogène,
(b) un radical de formule : 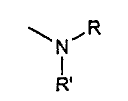 R et R' ayant la signification donnée ci-après,
(c) un radical -OR₁₂
(d) un reste de sucre ou d'aminoacide,
R₉, identique ou différent, représente un atome d'hydrogène ou un radical alkyle ayant de 1 à 6 atomes de carbone linéaire ou ramifié éventuellement substitué par un ou plusieurs atomes d'halogène,
R₁₀ et R₁₁, identiques ou différents, représentent un atome d'hydrogène ou un radical alkyle ayant de 1 à 6 atomes de carbone linéaire ou ramifié éventuellement substitué par un ou plusieurs atomes d'halogène,
R₁₂ représente un atome d'hydrogène, un radical alkyle ayant de 1 à 6 atomes de carbone linéaire ou ramifié éventuellement substitué par un ou plusieurs atomes d'halogène, un radical mono- ou polyhydroxyalkyle, un radical aryle ou aralkyle éventuellement substitué(s),
R et R', identiques ou différents, représentent des groupements protecteurs de fonctions amines, un atome d'hydrogène, un radical alkyle ayant de 1 à 6 atomes de carbone linéaire ou ramifié éventuellement substitué par un ou plusieurs atomes d'halogène ou encore, pris ensemble, forment un hétérocycle,
m est égal à 0 ou 1,
et les isomères optiques et géométriques desdits composés de formule (I) ainsi que leurs sels.

2. Composés selon la revendication 1, caractérisés par le fait qu'ils se présentent sous forme de sels d'un métal alcalin ou alcalino-terreux, de zinc, d'une amine organique, d'un acide minéral ou organique.

3. Composés selon l'une des revendications 1 ou 2, caractérisés par le fait que les radicaux alkyle ayant de 1 à 6 atomes de carbone linéaire ou ramifié éventuellement substitué par un ou plusieurs atomes d'halogène s sont choisis parmi les radicaux méthyle, éthyle, isopropyle, butyle, tertiobutyle et hexyle.

4. Composés selon l'une des revendications précédentes, caractérisés en ce que les radicaux alkényle ayant de 2 à 6 atomes de carbone linéaire ou ramifiéalkényles ayant de 2 à 6 atomes de carbone linéaires ou ramifiés correspondent à des radicaux allyle ou vinyle.

5. Composés selon l'une des revendications précédentes, caractérisés en ce que les radicaux alkynyles ayant de 3 à 6 atomes de carbone linéaires ou ramifiés correspondent au radical propargyle.

6. Composés selon l'une des revendications précédentes, caractérisés en ce que le radical acyle ayant de 1 à 6 atomes de carbone linéaire ou ramifié correspond au radical acétyle, propionyle ou pivaloyle.

7. Composés selon l'une des revendications précédentes, caractérisés en ce que les radicaux polyhydroxyalkyle sont choisis parmi les radicaux 2,3-dihydroxypropyle, 2,3,4-trihydroxybutyle, 2,3,4,5-tétrahydroxypentyle ou le reste du pentaérythritol..

8. Composés selon l'une des revendications précédentes, caractérisés en ce que les restes de sucre sont choisis dans le groupe constitué par les restes de glucose, de galactose, de mannose et d'acide glucuronique.

9. Composés selon l'une quelconque des revendications précédentes, caractérisés en ce que les restes d'aminoacide sont choisis dans le groupe constitué par les restes dérivant de la lysine, de la glycine ou de l'acide aspartique.

10. Composés selon l'une quelconque des revendications précédentes, caractérisés en ce que les radicaux polyéther sont choisis parmi les radicaux méthoxyméthyl éther, méthoxyéthoxyméthyl éther ou méthylthiométhyl éther.

11. Composés selon la revendication 1, caractérisés par le fait qu'ils sont pris, seuls ou en mélanges, dans le groupe constitué par :
3-[3-(5,6,7,8-tetrahydro-5,5,8,8-tetraméthyl-2-naphtyl)-3-méthyl-2,3-dihydro-benzofuran-5-yl]-acrylate de méthyle,
acide 3-[3-(5,6,7,8-tetrahydro-5,5,8,8-tetraméthyl-2-naphtyl)-3-méthyl-2,3-dihydro-benzofuran-5-yl]-acrylique,
acide [3-méthyl-3(5,5,8,8-tetraméthyl-5,6,7,8-tetrahydro-naphtalen-2-yl)-2,3-dihydro-benzofuran-5-yl]-propynoique,
(+)-3-[3-méthyl-3-(5,5,8,8-tetraméthyl-5,6,7,8-tetrahydro-naphtalen-2-yl)-2,3-dihydro-benzofuran-5-yl]-acrylate d'éthyle
acide (+)-3-[3-méthyl-3-(5,5,8,8-tetraméthyl-5,6,7,8-tetrahydro-naphtalen-2-yl)-2, 3-dihydro-benzofuran-5-yl]-acrylique
(-)-3-[3-méthyl-3-(5,5,8,8-tetraméthyl-5,6,7,8-tetrahydro-naphtalen-2-yl)-2,3-dihydro-benzofuran-5-yl]-acrylate d'éthyle
acide (-)-3-[3-Méthyl-3-(5,5,8,8-tetraméthyl-5,6,7,8-tetrahydro-naphtalen-2-yl)-2,3-dihydro-benzofuran-5-yl]-acrylique.
3-[3-méthyl-3-(3,5,5,8,8-pentaméthyl-5,6,7,8-tetrahydro-naphtalen-2-yl)-2,3-dihydro-benzofuran-5-yl]-acrylate d'éthyle
acide 3-[3-méthyl-3-(3,5,5,8,8-pentaméthyl-5,6,7,8-tetrahydro-naphtalen-2-yl)-2,3-dihydro-benzofuran-5-yl]-acrylique.
3-[3-méthyl-3-(naphtalen-2-yl)-2,3-dihydro-benzofuran-5-yl]-acrylate d'éthyle
acide 3-[3-méthyl-3-(naphtalen-2-yl)-2,3-dihydro-benzofuran-5-yl]-acrylique.
3-[3-(8,8-diméthyl-5,6,7,8-tetrahydro-naphtalen-2-yl)-3-méthyl-2,3-dihydro-benzofuran-5-yl]-acrylate d'éthyle
acide 3-[3-(8,8-diméthyl-5,6,7,8-tetrahydro-naphtalen-2-yl)-3-méthyl-2,3-dihydro-benzofuran-5-yl]-acrylique
3-[3-(5,5-diméthyl-5,6,7,8-tetrahydro-naphtalen-2-yl)-3-méthyl-2,3-dihydro-benzofuran-5-yl]-acrylate d'éthyle.
acide 3-[3-(5,5-diméthyl-5,6,7,8-tetrahydro-naphtalen-2-yl)-3-méthyl-2,3-dihydro-benzofuran-5-yl]-acrylique.
3-[3-méthyl-3-(5,5,8,8-tetraméthyl-5,6,7,8-tetrahydro-naphtalen-2-yl)-2,3-dihydro-benzofuran-6-yl]-acrylate d'éthyle
acide 3-[3-méthyl-3-(5,5,8,8-tetraméthyl-5,6,7,8-tetrahydro-naphtalen-2-yl)-2,3-dihydro-benzofuran-6-yl]-acrylique.
3-[3-allyl-3-(5,5,8,8-tetraméthyl-5,6,7,8-tetrahydro-naphtalen-2-yl)-2,3-dihydro-benzofuran-5-yl]-acrylaté d'éthyle
acide 3-[3-allyl-3-(5,5,8,8-tetraméthyl-5,6,7,8-tetrahydro-naphtalen-2-yl)-2,3-dihydro-benzofuran-5-yl]-acrylique
(E)-3-[3-méthyl-3-(5,5,8,8-tetraméthyl-5,6,7,8-tetrahydro-naphtalen-2-yl)-2,3-dihydro-benzofuran-5-yl]-but-2-enaote de méthyle
(Z)-3-[3-méthyl-3-(5,5,8,8-tetraméthyl-5,6,7,8-tetrahydro-naphtalen-2-yl)-2,3-dihydro-benzofuran-5-yl]-but-2-enoate de méthyle.
acide (E)-3-[3-méthyl-3-(5,5,8,8-tetraméthyl-5,6,7,8-tetrahydro-naphtalen-2-yl)-2,3-dihydro-benzofuran-5-yl]-but-2-enoique
3-[3-méthyl-3-(5,5,8,8-tetraméthyl-5,6,7,8-tetrahydro-naphtalen-2-yl)-2,3-dihydro-benzo[*b*]thiophen-5-yl]-acrylate d'éthyle.
acide 3-[3-méthyl-3-(5,5,8,8-tetraméthyl-5,6,7,8-tetrahydro-naphtalen-2-yl)-2,3-dihydro-benzo[*b*]thiophen-5-yl]-acrylique.
3-[3-(5,5,7,8-tetrahydro-5,5,8,8-tetraméthyl-2-naphtyl)-7-méthoxy-3-méthyl-2,3-dihydro-benzofuran-5-yl]-acrylate d'éthyle
acide 3-[3-(5,6,7,8-tetrahydro-5,5,8,8-tetraméthyl-2-naphtyl)-7-méthoxy-3-méthyl-2,3-dihydro-benzofuran-5-yl]-acrylique.
*N*-(4-hydroxy-phenyl)-3-[7-methoxy-3-méthyl-3-(5,5,8,8-tetraméthyl-5,5,7,8-tetrahydro-naphtalen-2-yl)-2,3-dihydro-benzofuran-5-yl]-acrylamide.
acide 3-[3-méthyl-3-(5,5,8,8-tetraméthyl-5,6,7,8-tetrahydro-naphtalen-2-yl)-2,3-dihydro-*1H*-indol-5-yl]-acrylique.
3-[3-méthyl-3-(5,5,8,8-tetraméthyl-5,6,7,8-tetrahydro-naphtalen-2-yl)-2,3-dihydro-*1H*-indol-5-yl]-acrylate de méthyle.

12. Composés selon la revendication 1, caractérisés par le fait qu'ils présentent l'une au moins des caractéristiques suivantes :
- R₁ et R₂ , pris ensemble, forment un cycle aromatique tel que décrit dans la revendication 1,
- R₃ est un hydrogène, un radical alkényle ayant de 2 à 6 atomes de carbone linéaire ou ramifié, un radical alkyle ayant de 1 à 6 atomes de carbone linéaire ou ramifié éventuellement substitué par un ou plusieurs atomes d'halogène ou un radical -OR₇
- R₄ est un hydrogène,
- R₅ est un radical de formule (i) ou (iii),
- R₆ est un hydrogène,
- R₈ est un radical OR₁₂,
- X et Y représentent CH,
- Z₁ est un atome d'oxygène ou de soufre,

13. Composés selon l'une quelconque des revendications précédentes pour une utilisation comme médicament.

14. Utilisation d'un composé selon la revendication 13 pour la préparation d'un médicament destiné au traitement
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle;
- d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal);
- des affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale;
- desaffections inflammatoires ne présentant pas de trouble de la kératinisation;
- des proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires;
- des désordres dermatologiques tels que les dermatoses bulleuses et les maladies du collagène;
- des troubles ophtalmologiques, notamment les coméopathies;
- du vieillissement de la peau, qu'il soit photoinduit ou chronologique,des pigmentations et des kératoses actiniques, ou toutes pathologies associées au vieillissement chronologique ou actinique;
- des stigmates de l'atrophie épidermique et/ou dermique induite par les corticostéroïdes locaux ou systémiques, ou tout autre forme d'atrophie cutanée,
- des troubles de la cicatrisation ou des vergetures;
- des troubles de la fonction sébacée tels que l'hyperséborrhée de l'acné ou la séborrhée simple;
- des états cancéreux ou précancéreux, plus particuliérement les leucémies promyélocytaires;
- d'affections inflammatoires telles que l'arthrite,
- de toute affection d'origine virale au niveau cutané ou général;
- de l'alopécie;
- d'affections dermatologiques à composante immunitaire;
- d'affections du système cardiovasculaire telles que l'artériosclérose ou l'hypertension ainsi que le diabète non-insulino dépendant,
- de désordres cutanés dus à une exposition aux rayonnements U.V..

15. Composition pharmaceutique, caractérisée par le fait qu'elle comprend, dans un support pharmaceutiquement acceptable, au moins l'un des composés tels que définis à l'une quelconque des revendications 1 à 12.

16. Composition selon la revendication 15, caractérisée en ce que la concentration en composé(s) selon l'une des revendications 1 à 12 est comprise entre 0,001 % et 5 % en poids par rapport à l'ensemble de la composition.

17. Composition cosmétique, caractérisée par le fait qu'elle comprend, dans un support cosmétiquement acceptable, au moins l'un des composés tels que définis à l'une quelconque des revendications 1 à 12.

18. Composition selon la revendication 17, caractérisée en ce que la concentration en composé(s) selon l'une des revendications 1 à 12 est comprise entre 0,001 % et 3 % en poids par rapport à l'ensemble de la composition.

19. Utilisation d'une composition cosmétique telle que définie à l'une des revendications 17 ou 18 pour l'hygiène corporelle ou capillaire.

## Claims

1. Compounds, characterized by the fact that they correspond to the following general formula (I) : 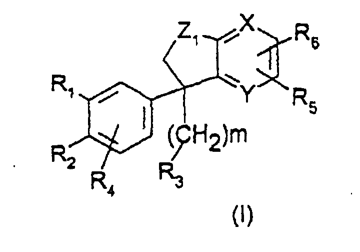 in which:
Z₁ is an atom or a radical chosen from: O, S and NR',
X and Y, which are identical or different, are CH or N, it being understood that X and Y cannot simultaneously be nitrogen atoms,
R₁ and R₂ taken together form, with the adjacent aromatic ring, a ring with 5 or 6 members which is optionally substituted by methyl groups and/or optionally interrupted by an SO radical, an SO₂ radical, or an oxygen or sulphur atom,
R₃ is:
(i) a hydrogen atom, a linear or branched alkyl radical having from 1 to 6 carbon atoms; optionally substituted by one or more halogen atoms, a linear or branched alkenyl radical having from 2 to 6 carbon atoms, a linear or branched alkynyl radical having from 3 to 6 carbon atoms, an aryl radical, a monohydroxyalkyl or polyhydroxyalkyl radical, a polyether radical, a cyano radical or an -O-R₇ radical,
R₇ having the meaning given below,
(ii) a radical of formula: 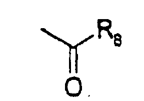 R₈ having the meaning given below,
or (iii) a radical of formula: 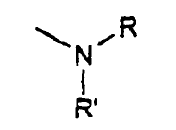 R and R' having the meaning given below
R₄ is:
(i) a hydrogen atom,
(ii) a linear or branched alkyl radical having from 1 to 6 carbon atoms, optionally substituted by one or more halogen atoms
(iii) a halogen atom,
(iv) an -OR₇ radical,
R₇ having the meaning given below,
R₅ is:
(i) a radical of formula: 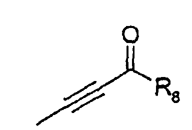
(ii) a radical of formula: 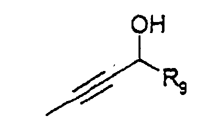
(iii) a radical of formula: 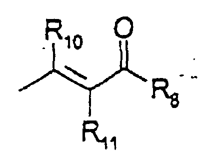
(iv) a radical of formula: 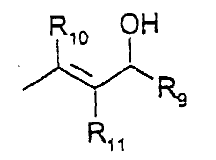
(v) a radical of formula: 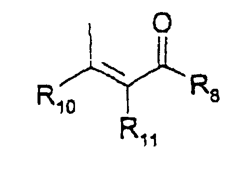
(vi) a radical of formula: 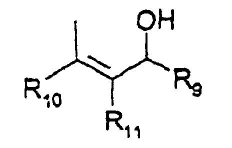 R₈, R₉, R₁₀ and R₁₁ having the meanings given below,
R₆ is a hydrogen atom, a halogen atom, a linear or branched alkyl radical having from 1 to 6 carbon atoms, optionally substituted by one or more halogen atoms, or the -OR₇ radical,
R₇ having the meaning given below, R₇, which is identical or different, is a hydrogen atom, a linear or branched alkyl radical having from 1 to 6 carbon atoms, optionally substituted by one or more halogen atoms, an aryl radical, an aralkyl radical, optionally substituted, a monohydroxyalkyl or polyhydroxyalkyl radical, a polyether radical or a linear or branched acyl radical having from 1 to 6 carbon atoms,
R₈ is:
(a) a hydrogen atom, a linear or branched lower alkyl radical having from 1 to 6 carbon atoms, optionally substituted by one or more halogen atoms,
(b) a radical of formula: 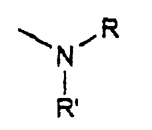 R and R' having the meaning given below,
(c) an -OR₁₂ radical
(d) a sugar or amino acid residue,
R₉, which is identical or different, is a hydrogen atom or a linear or branched alkyl radical having from 1 to 6 carbon atoms, optionally substituted by one or more halogen atoms,
R₁₀ and R₁₁, which are identical or different, are a hydrogen atom or a linear or branched alkyl radical having from 1 to 6 carbon atoms, optionally substituted by one or more halogen atoms,
R₁₂ is a hydrogen atom, a linear or branched alkyl radical having from 1 to 6 carbon atoms, optionally substituted by one or more halogen atoms, a mono- or polyhydroxyalkyl radical, an optionally substituted aryl or aralkyl radical,
R and R', which are identical or different, are protective groups of amine functions, a hydrogen atom, a linear or branched alkyl radical having from 1 to 6 carbon atoms, optionally substituted by one or more halogen atoms, or alternatively, taken together, form a heterocycle,
m is equal to 0 or 1,
and the optical and geometric isomers of the said compounds of formula (I) as well as their salts.

2. Compounds according to Claim 1, characterized by the fact that they are present in the form of salts of an alkali or alkaline earth metal, of zinc, of an organic amine, or of a mineral or organic acid.

3. Compounds according to either of Claims 1 or 2, characterized by the fact that the linear or branched alkyl radicals having from 1 to 6 carbon atoms, optionally substituted by one or more halogen atoms, are chosen from methyl, ethyl, isopropyl, butyl, tertiary butyl and hexyl radicals.

4. Compounds according to one of the preceding claims, characterized in that the linear or branched alkenyl radicals having from 2 to 6 carbon atoms correspond to allyl or vinyl radicals.

5. Compounds according to one of the preceding claims, characterized in that the linear or branched alkynyl radicals having from 3 to 6 carbon atoms correspond to the propargyl radical.

6. Compounds according to one of the preceding claims, characterized in that the linear or branched acyl radical having from 1 to 6 carbon atoms corresponds to the acetyl, propionyl or pivaloyl radical.

7. Compounds according to one of the preceding claims, characterized in that the polyhydroxyalkyl radicals are chosen from the 2,3-dihydroxypropyl, 2,3,4-trihydroxybutyl and 2,3,4,5-tetrahydroxypentyl radicals or the pentaerythritol residue.

8. Compounds according to one of the preceding claims, characterized in that the sugar residues are chosen from the group formed by the glucose, galactose, mannose and glucuronic acid residues.

9. Compounds according to any one of the preceding claims, characterized in that the amino acid residues are chosen from the group formed by the residues derived from lysine, glycine or aspartic acid.

10. Compounds according to any one of the preceding claims, characterized in that the polyether radicals are chosen from the methoxymethyl ether, methoxyethoxymethyl ether or methylthiomethyl ether radicals.

11. Compounds according to Claim 1, characterized by the fact that they are taken, alone or as a mixture, from the group formed by:
methyl 3-[3-(5,6,7,8-tetrahydro-5,5,8,8-tetramethyl-2-naphthyl)-3-methyl-2,3-dihydrobenzofuran-5-yl]acrylate,
3-[3-(5,6,7,8-tetrahydro-5,5,8,8-tetramethyl-2-naphthyl)-3-methyl-2,3-dihydrobenzofuran-5-yl]acrylic acid,
[3-methyl-3-(5,5,8,8-tetramethyl-5,6,7,8-tetrahydronaphthalen-2-yl)-2,3-dihydrobenzofuran-5-yl]propynoic acid,
ethyl (+)-3-[3-methyl-3-(5,5,8,8-tetramethyl-5,6,7,8-tetrahydronaphthalen-2-yl)-2,3-dihydrobenzofuran-5-yl]-acrylate,
(+)-3-[3-methyl-3-(5,5,8,8-tetramethyl-5,6,7,8-tetrahydronaphthalen-2-yl)-2,3-dihydrobenzofuran-5-yl]acrylic acid,
ethyl (-)-3-[3-methyl-3-(5,5,8,8-tetramethyl-5,6,7,8-tetrahydronaphthalen-2-yl)-2,3-dihydrobenzofuran-5-yl]-acrylate,
(-)-3-[3-methyl-3-(5,5,8,8-tetramethyl-5,6,7,8-tetra-hydronaphthalen-2-yl)-2,3-dihydrobenzofuran-5-yl]acrylic acid,
ethyl 3-[3-methyl-3-(3,5,5,8,8-pentamethyl-5,6,7,8-tetrahydronaphthalen-2-yl)-2,3-dihydrobenzofuran-5-yl]-acrylate,
3-[3-methyl-3-(3,5,5,8,8-pentamethyl-5,6,7,8-tetrahydronaphthalen-2-yl)-2,3-dihydrobenzofuran-5-yl]acrylic acid,
ethyl 3-[3-methyl-3-(naphthalen-2-yl)-2,3-dihydrobenzofuran-5-yl]acrylate,
3-[3-methyl-3-(naphthalen-2-yl)-2,3-dihydrobenzofuran-5-yl]acrylic acid,
ethyl 3-[3-(8,8-dimethyl-5,6,7,8-tetrahydronaphthalen-2-yl)-3-methyl-2,3-dihydrobenzofuran-5-yl]acrylate,
3-[3-(8,8-dimethyl-5,6,7,8-tetrahydronaphthalen-2-yl)-3-methyl-2,3-dihydrobenzofuran-5-yl]acrylic acid,
ethyl 3-[3-(5,5-dimethyl-5,6,7,8-tetrahydronaphthalen-2-yl)-3-methyl-2,3-dihydrobenzofuran-5-yl]acrylate,
3-[3-(5,5-dimethyl-5,6,7,8-tetrahydronaphthalen-2-yl)-3-methyl-2,3-dihydrobenzofuran-5-yl]acrylic acid,
ethyl 3-[3-methyl-3-(5,5,8,8-tetramethyl-5,6,7,8-tetrahydronaphthalen-2-yl)-2,3-dihydrobenzofuran-6-yl]-acrylate,
3-[3-methyl-3-(5,5,8,8-tetramethyl-5,6,7,8-tetrahydronaphthalen-2-yl)-2,3-dihydrobenzofuran-6-yl]acrylic acid,
ethyl 3-[3-allyl-3-(5,5,8,8-tetramethyl-5,6,7,8-tetrahydronaphthalen-2-yl)-2,3-dihydrobenzofuran-5-yl]-acrylate,
3-[3-allyl-3-(5,5,8,8-tetramethyl-5,6,7,8-tetrahydronaphthalen-2-yl)-2,3-dihydrobenzofuran-5-yl]acrylic acid,
methyl (E)-3-[3-methyl-3-(5,5,8,8-tetramethyl-5,6,7,8-tetrahydronaphthalen-2-yl)-2,3-dihydrobenzofuran-5-yl]-but-2-enaote,
methyl (Z)-3-[3-methyl-3-(5,5,8,8-tetramethyl-5,6,7,8-tetrahydronaphthalen-2-yl)-2,3-dihydrobenzofuran-5-yl]-but-2-enoate,
(E) -3-[3-methyl-3-(5,5,8,8-tetramethyl-5,6,7,8-tetrahydronaphthalen-2-yl)-2,3-dihydrobenzofuran-5-yl] but-2-enoic acid,
ethyl 3-[3-methyl-3-(5,5,8,8-tetramethyl-5,6,7,8-tetrahydronaphthalen-2-yl)-2,3-dihydrobenzo[*b*]thiophen-5-yl]-acrylate,
3-[3-methyl-3-(5,5,8,8-tetramethyl-5,6,7,8-tetrahydronaphthalen-2-yl)-2,3-dihydrobenzo[*b*]thiophen-5-yl]-acrylic acid,
ethyl 3-[3-(5,6,7,8-tetrahydro-5,5,8,8-tetramethyl-2-naphthyl)-7-methoxy-3-methyl-2,3-dihydrobenzofuran-5-yl]acrylate,
3-[3-(5,6,7,8-tetrahydro-5,5,8,8-tetramethyl-2-naphthyl)-7-methoxy-3-methyl-2,3-dihydrobenzofuran-5-yl]acrylic acid,
*N*-(4-hydroxyphenyl)-3-[7-methoxy-3-methyl-3-(5,5,8,8-tetramethyl-5,6,7,8-tetrahydronaphthalen-2-yl)-2,3-dihydrobenzofuran-5-yl]acrylamide,
3-[3-methyl-3-(5,5,8,8-tetramethyl-5,6,7,8-tetrahydronaphthalen-2-yl)-2,3-dihydro-*1H*-indol-5-yl]acrylic acid, methyl 3-[3-methyl-3-(5,5,8,8-tetramethyl-5,6,7,8-tetrahydronaphthalen-2-yl)-2,3-dihydro-*1H*-indol-5-yl]acrylate.

12. Compounds according to Claim 1, characterized by the fact that they have at least one of the following characteristics:
- R₁ and R₂, taken together, form an aromatic ring such as described in Claim 1,
- R₃ is a hydrogen, a linear or branched alkenyl radical having from 2 to 6 carbon atoms, a linear or branched alkyl radical having from 1 to 6 carbon atoms, optionally substituted by one or more halogen atoms, or an -OR₇ radical
- R₄ is a hydrogen,
- R₅ is a radical of formula (i) or (iii),
- R₆ is a hydrogen,
- R₈ is an OR₁₂ radical,
- X and Y are CH,
- Z₁ is an oxygen or sulphur atom.

13. Compounds according to any one of the preceding claims for use as a medicament.

14. Use of a compound according to Claim 13 for the preparation of a medicament intended for the treatment
- of dermatological conditions connected with a keratinization disorder bearing on differentiation and on proliferation, especially acne vulgaris, comedonian acne, polymorphic acne, acne rosacea, nodulocystic acne, acne conglobata, senile acne, secondary acne such as solar acne, acne medicamentosa or occupational acne;
- of other types of keratinization disorders, especially ichthyosis, ichthyosiform states, Darier's disease, keratosis palmaris and plantaris, leucoplakias and leucoplakiform states, cutaneous or mucous (buccal) lichen;
- of dermatological conditions connected with a keratinization disorder with an inflammatory and/or immunoallergic component and especially all the forms of psoriasis whether it is cutaneous, mucous or ungual, and even arthropathic psoriasis, or alternatively cutaneous atopy, such as eczema or respiratory atopy or alternatively gingival hypertrophy;
- of inflammatory conditions not presenting a keratinization disorder;
- of dermal or epidermal proliferations whether they are benign or malignant, whether or not they are of a viral origin such as verruca vulgaris, verruca plana and epidermodysplasia verruciformis, oral or florid papillomatosis and proliferations able to be induced by ultra-violet, especially in the case of basal and spinocellular epithelioma;
- of dermatological disorders such as bullosis and collagen diseases;
- of ophthalmological disorders, especially corneopathies;
- of ageing of the skin, whether it is photoinduced or chronological, pigmentation and actinic keratosis, or any pathologies associated with chronological or actinic aging;
- of stigmata of epidermal and/or dermal atrophy induced by local or systemic corticosteroids, or any other form of cutaneous atrophy,
- of cicatrization disorders or vibices; sebaceous function disorders such as hyperseborrhoea of acne or simple seborrhoea;
- of cancerous or precancerous states, more partïcularly promyelocytic leukaemias;
- of inflammatory disorders such as arthritis,
- of any disorder of viral origin at the cutaneous or general level;
- of alopecia;
- of dermatological disorders with an immunological component;
- of disorders of the cardiovascular system such as arteriosclerosis or hypertension as well as non-insulin dependent diabetes,
- of cutaneous disorders due to exposure to U.V. rays.

15. Pharmaceutical composition, characterized by the fact that it comprises, in a pharmaceutically acceptable support, at least one of the compounds as defined in any one of Claims 1 to 12.

16. Composition according to Claim 15, characterized in that the concentration of compound(s) according to one of Claims 1 to 12 is between 0.001% and 5% by weight with respect to the whole of the composition.

17. Cosmetic composition, characterized by the fact that it comprises, in a cosmetically acceptable support, at least one of the compounds as defined in any of Claims 1 to 12.

18. Composition according to Claim 17, characterized in that the concentration of compound(s) according to one of Claims 1 to 12 is between 0.001% and 3% by weight with respect to the whole of the composition.

19. Use of a cosmetic composition as defined in either of Claims 17 or 18 for body or hair hygiene.

## Patentansprüche

1. Verbindungen, dadurch gekennzeichnet, daß sie die folgende allgemeine Formel (I) 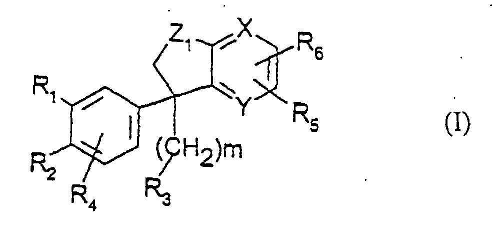 aufweisen, in der bedeuten:
- Z₁ ein Atom oder eine Gruppe, das/die unter O, S und NR' ausgewählt ist,
- X und Y, die gleich oder verschieden sind, CH oder N, mit der Maßgabe, daß X und Y nicht gleichzeitig Stickstoffatome bedeuten können,
- R₁ und R₂ zusammen genommen Reste, die mit dem benachbarten aromatischen Ring einen Ring mit 5 oder 6 Kettengliedern bilden, der gegebenenfalls mit Methylgruppen substituiert ist und/oder gegebenenfalls durch eine Gruppe SO, SO₂, ein Sauerstoffatom oder ein Schwefelatom unterbrochen ist,
- R₃
(i) ein Wasserstoffatom, geradkettiges oder verzweigtes C₁-C₆-Alkyl, das gegebenenfalls mit einem oder mehreren Halogenatomen substituiert ist, geradkettiges oder verzweigtes C₂-C₆-Alkenyl, geradkettiges oder verzweigtes C₃-C₆-Alkinyl, Aryl, Monohydroxyalkyl oder Polyhydroxyalkyl, einen Polyetherrest, Cyano oder eine Gruppe -O-R₇, worin R₇ die weiter unten angegebene Bedeutung hat,
(ii) eine Gruppe der Formel 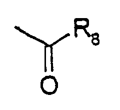 , worin R₈ die weiter unten angegebene Bedeutung hat, oder
(iii) eine Gruppe der Formel 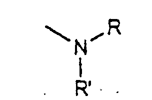 , worin R und R' die weiter unten angegebene Bedeutung haben,
- R₄
(i) ein Wasserstoffatom,
(ii) geradkettiges oder verzweigtes C₁-C₆-Alkyl, das gegebenenfalls mit einem oder mehreren Halogenatomen substituiert ist,
(iii) ein Halogenatom,
(iv) eine Gruppe -OR₇, worin R₇ die weiter unten angegebene Bedeutung hat.
- R₅
(i) eine Gruppe der Formel 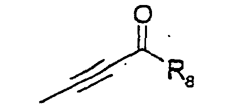
(ii) eine Gruppe der Formel 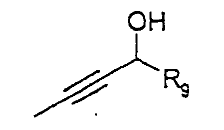
(iii) eine Gruppe der Formel 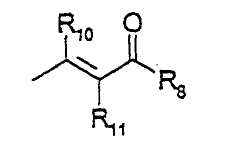
(iv) eine Gruppe der Formel 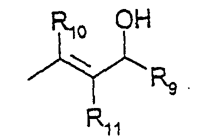
(v) eine Gruppe der Formel 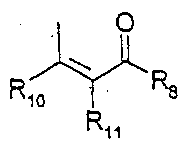
(vi) eine Gruppe der Formel 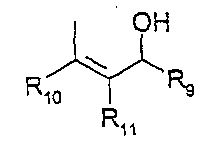 worin R₈, R₉, R₁₀ und R₁₁ die weiter unten angegebene Bedeutung haben,
- R₆ ein Wasserstoffatom, ein Halogenatom, geradkettiges oder verzweigtes C₁-C₆-Alkyl, das gegebenenfalls mit einem oder mehreren Halogenatomen substituiert ist, oder die Gruppe -O-R₇, worin R₇ die weiter unten angegebene Bedeutung hat,
- R₇, gleich oder verschieden, ein Wasserstoffatom, geradkettiges oder verzweigtes C₁-C₆-Alkyl, das gegebenenfalls mit einem oder mehreren Halogenatomen substituiert ist, Aryl, Aralkyl, Monohydroxyalkyl oder Polyhydroxyalkyl, einen Polyetherrest oder eine geradkettige oder verzweigte C₁-C₆-Acylgruppe,
- R₈:
(a) ein Wasserstoffatom, geradkettiges oder verzweigtes C₁-C₆-Alkyl, das gegebenenfalls mit einem oder mehreren Halogenatomen substituiert ist,
(b) einen Rest der Formel 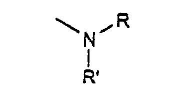 worin R und R' die weiter unten angegebene Bedeutung haben,
(c) eine Gruppe -OR₁₂,
(d) einen Zucker- oder Aminosäurerest,
- R₉, gleich oder verschieden, ein Wasserstoffatom, geradkettiges oder verzweigtes C₁-C₆-Alkyl, das gegebenenfalls mit einem oder mehreren Halogenatomen substituiert ist,
- R₁₀ und R₁₁, gleich oder verschieden, ein Wasserstoffatom, geradkettiges oder verzweigtes C₁-C₆-Alkyl, das gegebenenfalls mit einem oder mehreren Halogenatomen substituiert ist,
- R₁₂ ein Wasserstoffatom, geradkettiges oder verzweigtes C₁-C₆-Alkyl, das gegebenenfalls mit einem oder mehreren Halogenatomen substituiert ist, Mono- oder Polyhydroxyalkyl, Aryl oder Aralkyl, das/die gegebenenfalls substituiert ist/sind,
- R und R', gleich oder verschieden, Schutzgruppen für Amingruppen, ein Wasserstoffatom, geradkettiges oder verzweigtes C₁-C₆-Alkyl, das gegebenenfalls mit einem oder mehreren Halogenatomen substituiert ist, oder zusammen genommen Reste, die einen Heterocyclus bilden,
- m die ganze Zahl 0 oder 1,
und die optischen und geometrischen Isomere dieser Verbindungen der Formel (I) sowie ihre Salze.

2. Verbindungen nach Anspruch 1, dadurch gekennzeichnet, daß sie in Form eines Alkalimetall- oder Erdalkalimetallsalzes, des Zinksalze, eines Salzes eines organischen Amins, einer anorganischen Säure oder einer organischen Säure vorliegen.

3. Verbindungen nach einem der Ansprüche 1 und 2, dadurch gekennzeichnet, daß die geradkettigen oder verzweigten C₁-C₆-Alkylreste, die gegebenenfalls mit einem oder mehreren Halogenatomen substituiert sind, unter Methyl, Ethyl, Isopropyl, Butyl, tert.-Butyl und Hexyl ausgewählt sind.

4. Verbindungen nach einem der vorhergehenden Ansprüche, dadurch gekennzeichnet, daß die geradkettigen oder verzweigten C₂-C₆-Alkenylreste dem Allylrest oder dem Vinylrest entsprechen.

5. Verbindungen nach einem der vorhergehenden Ansprüche, dadurch gekennzeichnet, daß die geradkettigen oder verzweigten C₃-C₆-Alkinylreste dem Propargylrest entsprechen.

6. Verbindungen nach einem der vorhergehenden Ansprüche, dadurch gekennzeichnet, daß der geradkettige oder verzweigte C₁-C₆-Acetylrest dem Acetyl-, Propionyl- oder Pivaloylrest entspricht.

7. Verbindungen nach einem der vorhergehenden Ansprüche, dadurch gekennzeichnet, daß die Polyhydroxyalkylreste unter 2-3-Dihydroxypropyl, 2,3,4-Trihydroxybutyl, 2,3,4,5-Tetrahydroxypentyl und dem Pentaerythrit-Rest ausgewählt sind.

8. Verbindungen nach einem der vorhergehenden Ansprüche, dadurch gekennzeichnet, daß die Zuckerreste unter dem Glucose-, Galactose-, Mannose- und Glucuronsäure rest ausgewählt sind.

9. Verbindungen nach einem der vorhergehenden Ansprüche, dadurch gekennzeichnet, daß die Aminosäuregruppen unter den Gruppen ausgewählt sind, die von Lysin, Glycin und der Asparaginsäure abgeleitet sind.

10. Verbindungen nach einem der vorhergehenden Ansprüche, dadurch gekennzeichnet, daß die Polyether-Gruppen unter der Methoxymethylether-, der Methoxyethoxymethylether- und der Methylthiomethylether-Gruppe ausgewählt sind.

11. Verbindungen nach Anspruch 1, dadurch gekennzeichnet, daß sie einzeln oder im Gemisch aus der folgenden Gruppe entnommen werden, die aus den folgenden Verbindungen besteht:
3-[3-(5,6,7,8-Tetrahydro-5,5,8,8-tetramethyl-2-naphthyl)-3-methyl-2,3-dihydrobenzofuran-5-yl]-acrylsäuremethylester,
3-[3-(5,6,7,8-Tetrahydro-5,5,8,8-tetramethyl-2-naphthyl)-3-methyl-2,3-dihydrobenzofuran-5-yl]-acrylsäure,
[3-Methyl-3-(5,5,8,8-tetramethyl-5,6,7,8-tetrahydronaphthalin-2-yl)-2,3-dihydrobenzofuran-5-yl]-propinsäure,
(+)-3-[3-Methyl-3-(5,5,8,8-tetramethyl-5,6,7,8-tetrahydronaphthalin-2-yl)-2,3-dihydrobenzofuran-5-yl]-acrylsäureethylester,
(+)-3-[3-Methyl-3-(5,5,8,8-tetramethyl-5,6,7,8-tetrahydronaphthalin-2-yl)-2,3-dihydrobenzofuran-5-yl]-acrylsäure,
(-)-3-[3-Methyl-3-(5,5,8,8-tetramethyl-5,6,7,8-tetrahydronaphthalin-2-yl)-2,3-dihydrobenzofuran-5-yl]-acrylsäureethylester,
(-)-3-[3-Methyl-3-(5,5,8,8-tetramethyl-5,6,7,8-tetrahydronaphthalin-2-yl)-2,3-dihydrobenzofuran-5-yl]-acrylsäure,
3-[3-Methyl-3-(3,5,5,8,8-pentamethyl-5,6,7,8-tetrahydronaphthalin-2-yl)-2,3-dihydrobenzofuran-5-yl]-acrylsäureethylester,
3-[3-Methyl-3-(3,5,5,8,8-pentamethyl-5,6,7,8-tetrahydronaphthalin-2-yl)-2,3-dihydrobenzofuran-5-yl]-acrylsäure,
3-[3-Methyl-3-(naphthalin-2-yl)-2,3-dihydrobenzofuran-5-yl]-acrylsäureethylester,
3-[3-Methyl-3-(naphthalin-2-yl)-2,3-dihydrobenzofuran-5-yl]-acrylsäure,
3-[3-(8,8-Dimethyl-5,6,7,8-tetrahydronaphthalin-2-yl)-3-methyl-2,3-dihydrobenzofuran-5-yl]-acrylsäureethylester,
3-[3-(8,8-Dimethyl,5,6,7,8-tetrahydronaphthalin-2-yl)-3-methyl-2,3-dihydrobenzofuran-5-yl]-acrylsäure,
3-[3-(5,5-Dimethyl-5,6,7,8-tetrahydronaphthalin-2-yl)-3-methyl-2,3-dihydrobenzofuran-5-yl]-acrylsäureethylester,
3-[3-(5,5-Dimethyl-5,6,7,8-tetrahydronaphthalin-2-yl)-3-methyl-2,3-dihydrobenzofuran-5-yl]-acrylsäure,
3-[3-Methyl-3-(5,5,8,8-tetramethyl-5,6,7,8-tetrahydronaphthalin-2-yl)-2,3-dihydrobenzofuran-6-yl]-acrylsäureethylester,
3-[3-Methyl-3-(5,5,8,8-tetramethyl-5,6,7,8-tetrahydronaphthalin-2-yl)-2,3-dihydrobenzofuran-6-yl]-acrylsäure,
3-[3-Allyl-3-(5,5,8,8-tetramethyl-5,6,7,8-tetrahydronaphthalin-2-yl)-2,3-dihydrobenzofuran-5-yl]-acrylsäureethylester,
3-[3-Allyl-3-(5,5,8,8-tetramethyl-5,6,7,8-tetrahydronaphthalin-2-yl)-2,3-dihydrobenzofuran-5-yl]-acrylsäure,
(E)-3-[3-Methyl-3-(5,5,8,8-tetramethyl-5,6,7,8-tetrahydronaphthalin-2-yl)-2,3-dihydrobenzofuran-5-yl]-but-2-ensäuremethylester,
(Z)-3-[3-Methyl-3-(5,5,8,8-tetramethyl-5,6,7,8-tetrahydronaphthalin-2-yl)-2,3-dihydrobenzofuran-5-yl]-but-2-ensäuremethylester,
(E)-3-[3-Methyl-3-(5,5,8,8-tetramethyl-5,6,7,8-tetrahydronaphthalin-2-yl)-2,3-dihydrobenzofuran-5-yl]-but-2-ensäure,
3-[3-Methyl-3-(5,5,8,8-tetramethyl-5,6,7,8-tetrahydronaphthalin-2-yl)-2,3-dihydrobenzo[b]thiophen-5-yl]-acrylsäureethylester,
3-[3-Methyl-3-(5,5,8,8-tetramethyl-5,6,7,8-tetrahydronaphthalin-2-yl)-2,3-dihydrobenzo[b]thiophen-5-yl]-acrylsäure,
3-[3-(5,6,7,8-Tetrahydro-5,5,8,8-tetramethyl-2-naphthyl)-7-methoxy-3-methyl-2,3-dihydrobenzofuran-5-yl]-acrylsäureethylester,
3-[3-(5,6,7,8-Tetrahydro-5,5,8,8-tetramethyl-2-naphthyl)-7-methoxy-3-methyl-2,3-dihydrobenzofuran-5-yl]-acrylsäure,
*N*-(4-Hydroxyphenyl)-3-[7-methoxy-3-methyl-3-(5,5,8,8-tetramethyl-5,6,7,8-tetrahydronaphthalin-2-yl)-2,3-dihydrobenzofuran-5-yl]-acrylamid,
3-[3-Methyl-3-(5,5,8,8-tetramethyl-5,6,7,8-tetrahydronaphthalin-2-yl)-2,3-dihydro-*1H-*indol-5-yl]-acrylsäure,
3-[3-Methyl-3-(5,5,8,8-tetramethyl-5,6,7,8-tetrahydronaphthalin-2-yl)-2,3-dihydro-*1H*-indol-5-yl]-acrylsäuremethylester.

12. Verbindungen nach Anspruch 1, dadurch gekennzeichnet, daß sie mindestens eine der folgenden Bedingungen erfüllen:
- R₁ und R₂ bilden wie in Anspruch 1 beschrieben zusammen einen aromatischen Ring,
- R₃ ist ein Wasserstoffatom, geradkettiges oder verzweigtes C₂-C₆-Alkenyl, geradkettiges oder verzweigtes C₁-C₆-Alkyl, das gegebenenfalls mit einem oder mehreren Halogenatomen substituiert ist, oder eine Gruppe -O-R₇,
- R₄ ist ein Wasserstoffatom,
- R₅ ist eine Gruppe der Formel (i) oder (iii),
- R₆ ist ein Wasserstoffatom,
- R₈ stellt eine Gruppe OR₁₂ dar,
- X und Y bedeuten CH,
- Z₁ ist ein Sauerstoffatom oder ein Schwefelatom.

13. Verbindungen nach einem der vorhergehenden Ansprüche zur Verwendung als Arzneimittel.

14. Verwendung einer Verbindung nach Anspruch 13 zur Herstellung eines Arzneimittels, das vorgesehen ist zur Behandlung
- von Hautkrankheiten, die mit einer Störung der Keratinisierung verbunden sind, die die Differenzierung und Zellproliferation betrifft, insbesondere zur Behandlung der Akne vulgaris, Akne comedonica, der polymorphen Akne, der Akne rosaceae, der nodulären Akne, der Akne conglobata, der altersbedingten Aknen, der als Nebenwirkung auftretenden Aknen, wie der Akne solaris, der medikamentenbedingten Akne oder der Akne professionalis,
- anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (buccal) (Lichen),
- anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger- und Zehennägel betreffen, und des psoriatischen Rheumas und der Hautatopien, wie Ekzemen, oder der respiratorischen Atopie oder auch der Hypertrophie des Zahnfleischs,
- von Entzündungen, die nicht mit einer Störung der Keratinisierung zusammenhängen,
- aller gutartigen oder bösartigen Wucherungen der Dermis oder Epidermis, die gegebenenfalls viralen Ursprungs sind, wie Verruca vulgaris, Verruca plana, Epidermodysplasia verruciformis, orale Papillomatose, Papillomatosis florida, und der Wucherungen, die durch UV-Strahlung hervorgerufen werden können, insbesondere des Epithelioma basocellulare und Epithelioma spinocellulare,
- anderer Hautkrankheiten, wie der Dermatitis bullosa und der das Kollagen betreffenden Krankheiten,
- bestimmter Augenkrankheiten, insbesondere der Hornhauterkrankungen,
- der lichtbedingten und der altersbedingten Hautalterung, der Pigmentierungen und der Keratosis actinica und aller Krankheiten, die mit der normalen Alterung oder der lichtbedingten Alterung zusammenhängen,
- von Wunden/Narben der Atrophien der Epidermis und/oder Dermis, die durch lokal oder systemisch angewendete Corticosteroide hervorgerufen werden und aller sonstigen Arten der Hautatrophie,
- von Störungen der Wundheilung und Schwangerschaftsstreifen,
- von Störungen der Talgproduktion, wie Hyperseborrhö bei Akne oder der einfachen Seborrhö,
- von krebsartigen Zuständen oder präkanzerogenen Zuständen, insbesondere der promyelozytären Leukämien,
- von Entzündungserkrankungen, wie Arthritis,
- aller virusbedingten Erkrankungen der Haut oder anderer Bereiche des Körpers,
- der Alopecie,
- von Hautkrankheiten mit einer immunologischen Komponente, von Herz-Kreislauf-Erkrankungen, wie Arteriosklerose oder Bluthochdruck, sowie des Insulin-unabhängigen Diabetes,
- von Hautproblemen, die durch UV-Strahlung hervorgerufen werden.

15. Pharmazeutische Zusammensetzung, dadurch gekennzeichnet, daß sie in einem pharmazeutisch akzeptablen Träger mindestens eine der Verbindungen, die wie in den Ansprüchen 1 bis 12 definiert sind, enthält.

16. Zusammensetzung nach Anspruch 15, dadurch gekennzeichnet, daß die Konzentration an Verbindung(en) nach einem der Ansprüche 1 bis 12 im Bereich von 0,001 bis 5 Gew.-%, bezogen auf das Gesamtgewicht der Zusammensetzung, liegt:

17. Kosmetische Zusammensetzung, dadurch gekennzeichnet, daß sie in einem kosmetisch akzeptablen Träger mindestens eine der wie in einem der Ansprüche 1 bis 12 definierten Verbindungen enthält.

18. Zusammensetzung nach Anspruch 17, dadurch gekennzeichnet, daß die Konzentration an Verbindung(en) nach einem der Ansprüche 1 bis 12 im Bereich von 0,001 bis 3 Gew.-%, bezogen auf das Gesamtgewicht der Zusammensetzung, liegt.

19. Verwendung einer wie in einem der Ansprüche 17 und 18 definierten kosmetischen Zusammensetzung für die Körperreinigung oder die Reinigung der Haare.
